# EUROPEAN PATENT APPLICATION

(11) **EP 2 078 756 A1**
(43) Date of publication of application: **15.07.2009**
(21) Application number: 07827821.5
(22) Date of filing: 26.09.2007
(51) Int. Cl.: C12Q 1/68, C12N 15/09, C12Q 1/04

(54) **DETECTION OF BACTERIUM BY UTILIZING DNAJ GENE AND USE THEREOF**

(30) Priority: 03.10.2006 JP 2006272329; 26.03.2007 JP 2007079059
(71) Applicant: Gifu University, Gifu-shi Gifu 501-1193 (JP); Amr, Inc., Gifu-shi Gifu 501-1111 (JP)
(72) Inventor: EZAKI, Takayuki, Gifu-shi, Gifu 501-1193 (JP); OHKUSU, Kiyofumi, Gifu-shi, Gifu 501-1193 (JP); YOSHIDA, Shigeru, Gifu-shi, Gifu 501-1111 (JP)
(74) Representative: Woods, Geoffrey Corlett
(86) International application number: PCT/JP2007/001042
(87) International publication number: WO 2008/041354

(57) **Abstract**

Disclosed is a method for detecting the species of a bacterium in a simple manner. Specifically disclosed is a method for detecting the species of a bacterium, which can detect at least one bacterial species selected from *Staphylococcus* species, Streptococcus species, *Klebsiella* species, *Escherichia* species, *Mycobacterium* species, *Legionella* species, *Vibrio* species, *Bacillus* species, *Neisseria* species, *Campylobacter* species, *Chlamydia* species, *Chlamydophila* species, *Mycoplasma* species, *Listeria* species, *Salmonella* species and *Yersinia* species, and which comprises the steps of: a) contacting a sample suspected to contain a nucleic acid derived from the bacterium species to be detected with a nucleic acid molecule capable of hybridizing with at least a part of a DnaJ gene derived from the bacterial species; ; and b) detecting the presence or absence of the hybridization between the nucleic acid molecule and the nucleic acid contained in the sample.

## Description

### Technical Field

The present invention relates to the detection of bacteria by utilizing the DnaJ gene, and the use thereof, and more specifically relates to a method of detecting bacteria by utilizing the DnaJ gene, nucleic acid molecules for use in this method, and use of such a method and such nucleic acid molecules in diagnosis and identification.

### Background Art

The detection and identification of various types of bacteria such as pathogenic bacteria has until now required the use of special reagents and expertise, and cultivation of the target bacteria using such special reagents and expertise. In such, growing pathogenic bacteria involves degrees of danger. Moreover, when a plurality of pathogens is thought to be present, cultivation has to be carried out under various conditions. Moreover, identifying the species of bacteria has required the careful investigation of, e.g., biochemical properties. Hence, the detection and identification of bacteria, particularly the detection and identification of pathogenic bacteria, have entailed some danger and also required considerable time and effort.

Meanwhile, 16S rDNA base sequencing has been employed as a technique for bacterial classification and identification. It is expected that, by employing the base sequence of such genes, bacteria and bacterial species will be detected and identified in a relatively short time.

However, with the growing accumulation of 16S rDNA base sequences, it has become apparent that in cases where at least a 98% similarity resides in the 16S rDNA base sequence between independent but related bacterial species, the species cannot be distinguished (Non-Patent Document 1). This led to the realization that bacterial species cannot be determined without carrying out a more complicated process of measuring the degree of similarity for all chromosomal genes (Non-Patent Document 2). Accordingly, a need for the collection of data on sequences having a higher degree of variation has grown therefor, and progresses are being made in gathering base sequence data on genes such as GyrB and ropB (Non-Patent Documents 3, 4 and 5).

One such housekeeping gene is the heat shock protein DnaJ. It has been reported that the DnaJ gene coding for DnaJ can be used to classify mycobacterium species (Non-Patent Document 6). Moreover, it has also been reported that polymorphism is common in the DnaJ gene of Mycobacterium avium serotypes (Non-Patent Document 7) and that, although only having a short sequence of 200 bases, the DnaJ gene is well preserved within a serotype yet differences in sequence are large in the species level within the same genus (Non-Patent Document 8). In addition, it has been reported that, in the genus Legionella, the DnaJ gene is preserved in the serotypes of L. pneumophlia, but has a high degree of polymorphism at the species level (Non-Patent Document 9).

At the front lines of disease prevention and treatment, a strong desire for the rapid and reliable detection and identification of pathogenic microorganisms in samples collected from food products and animals exists. The multiplex PCR process is useful for identifying pathogenic microorganisms. This process involves using a plurality of primer sets, each of which corresponds to a characteristic target nucleic acid in one of a plurality of types of pathogenic microorganisms, to carry out polymer chain reactions (PCR). In multiplex PCR, multiple types of primer are used to amplify the target nucleic acids within a single tube. A variety of investigations are being conducted on such multiplex PCR techniques (Patent Document Nos. 1 to 4). Multiplex PCR is highly useful for identifying pathogenic microorganisms such as viruses and bacteria in the treatment of infections. To identify a pathogenic microorganism, it is necessary to target a specific sequence in the genome of that organism.

Non-Patent Document 1: Fox, G.E. et al.: Int. J. Syst. Bacteriol. 42, 166-170 (1992).
Non-Patent Document 2: Ezaki, T. et al.: Int. J. Syst. Bacteriol. (1989); Amano, M. et al.: Microbiol. Immunol. 49, 255-263 (2005).
Non-Patent Document 3: Yamamoto, S and Harayama, S.: Appl. Environ. Microbiol. 61, 1104-1109 (1995).
Non-Patent Document 4: Yamamoto, S. and Harayama, S.: Int. J. Syst. Bacteriol. 46, 506-511 (1996).
Non-Patent Document 5: Cheunoy, W. et al.: Diagn. Microbio/. Infect. Dis. 51, 165-171 (2005).
Non-Patent Document 6: Takewaki, S. et al.: Int. J. Syst. Bacteriol. 44, 159-166 (1994).
Non-Patent Document 7: Victor, T.C., et al.: J. Med. Microbiol. 44, 332-3 3 9 (1996).
Non-Patent Document 8: Morita, Y. et al.: J. Med. Microbiol. 53: 813-817 (2004).
Non-Patent Document 9: Liu, H. et al.: Microbiol. Immunol. 47, 859-869 (2003).

### Disclosure of the Invention

In general, only when the base sequence in a given gene is both specific with respect to related microorganisms in other genera and also specific at the species level within the same genus, and when the base sequence is preserved to some degree at the strain level within a species, it is possible to detect or identify a specific species of organism using only that gene. That is, determining whether a specific gene can be used to detect and identify a specific species of microorganism required that the specific gene be comprehensively sequenced for a plurality of strains included within the specific species.

However, the DnaJ gene has been sequenced only for specific species and at the serotype level; sequence data at the strain level within these species has not been published. A comprehensive gene analysis as in the aforestated case has not been carried out for the DnaJ gene. As a result, whether the DnaJ gene has a specific base sequence that can be used alone to detect or identify a species has been unclear until as of today. In addition, because of polymorphism among the strains, it has been necessary to sequence a large number of strains in order to find the sequence preserved in the species.

Also, in the identification of pathogenic microorganisms such as bacteria, when multiple types (from 2 to 10 types) of primers are used at the same concentration as when those primers are individually used (about 0.2 µM to 0.8 µM) a primer competition arises; the efficiency of amplification decreases and the amount of amplified product diminishes, as a result of which the amplified product cannot be detected. Furthermore, when the primer concentration decreases, the efficiency of amplification decreases, as a result of which the amplified product likewise cannot be detected. Hence, the number of types of practical primers that can be combined at one time has been substantially about six types.

The specificity of the primers is important in carrying out multiplex PCR with multiple types of primers. In multiplex PCR for multiple types of pathogenic microorganisms, it is necessary to target sequences specific to the species of organisms. However, designing primers which can be used at the same time on a plurality of types of pathogenic microorganisms and amplify species-specific targets has been exceedingly difficult.

One object of the present invention may be to provide a method capable of easily detecting or identifying a bacterial species, and nucleic acid molecules for use therefor. Another object of the invention may be to provide a method for detecting or identifying a bacterial species at a high sensitivity, and nucleic acid molecules for use therefor. Yet another object of the invention may be to provide a method for detecting or identifying two or more bacterial species, and nucleic acid molecules for use therefor. A further object of the invention may be to provide a microbial amplification method utilizing multiplex PCR which is suitable for the rapid detection of microorganisms such as bacteria, and a kit or the like for the same.

The inventors have sequenced the DnaJ gene for 680 species and 1,250 strains. Upon comparing the results, they have found that, whereas the degree of similarity in the 16S rDNA gene sequence between species was more than 95%, the similarity in the DnaJ gene between species in substantially all genera was from 75 to 90%. In addition, from the sequence data at the strain level within the same species, the inventors have discovered that it is possible to extract a specific base sequence which enables the species to be identified from the DnaJ gene alone. These discoveries ultimately led to the present invention. That is, the present invention may provide the following techniques.

The invention may provide a process for detecting bacteria, in which one, two or more species of bacteria selected from among bacteria of the genera Staphylococcus, Streptococcus, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia are subject bacterial species being subjects of detection, the process including:
(a) a step of contacting a test sample which may contain nucleic acids of the subject bacterial species with nucleic acid molecules which hybridize with at least portions of a DnaJ genes in the subject bacterial species, and
(b) a step of detecting whether the nucleic acid molecules have hybridized with nucleic acids within the test sample.

In this invention, the step (a) is preferably a step in which the nucleic acid sample is simultaneously contacted with two or more types of nucleic acid molecules which hybridize with at least a portion of the DnaJ gene in each of the two or more bacterial species. Also, the nucleic acid molecules may include one or more species-specific base sequence extracted by aligning the base sequences of the DnaJ gene of strains belonging to the same species, or may include one or more base sequences substantially identical therewith. Moreover, the bacteria that is the subject of detection may be of one or more species selected from among Streptococcus pyogenes, Streptococcus agalactiae, Streptococcus pneumoniae, Staphylococcus aureus, Klebsiella pneumoniae, Escherichia coli, Mycobacterium tuberculosis, Mycoplasma pneumoniae, Mycoplasma genitarium, Vibrio cholerae, Vibrio parahaemolyticus, Vibrio vulnificus, Vibrio fluvialis, Vibrio alginolyticus, Neisseria meningitides, Neisseria gonorrhoae, Bacillus cereus, Chlamydia trachomatis, Chlamydophila pneumoniae and Campylobacter jejuni. Specific nucleic acid molecules may be used to detect the respective specific bacterial species.

The subject bacteria may be one or more species selected from among the genera Streptococcus, Staphylococcus, Klebsiella, Escherichia, Mycobacterium, Legionella, Chlamydia, Chlamydophila, Neisseria and Mycoplasma. These bacteria are causative organisms for pneumonia and/or pharyngitis. The bacteria may be any species selected from among Streptococcus pyogenes, Streptococcus agalactiae, Streptococcus pneumoniae, Staphylococcus aureus, Klebsiella pneumoniae, Escherichia coli, Mycobacterium tuberculosis, Legionella pneumophila, Chlamydia trachomatis, Chlamydophila pneumoniae, Neisseria gonorrhoae, Neisseria meningitides, and Mycoplasma pneumoniae, in which case the nucleic acid molecules may include one or more base sequences selected from among SEQ ID NOS: 1 to 42, SEQ ID NOS: 131 to 134 and SEQ ID NOS: 89 to 116, or one or more base sequences substantially identical thereto.

Alternatively, the subject bacteria may be one or more species selected from bacteria of the genera Vibrio, Campylobacter, Staphylococcus, Bacillus, Escherichia, Listeria, Salmonella and Yersinia. These bacteria are causative organisms for diarrhea and/or food poisoning. The bacteria may be any species selected from among Vibrio cholerae, Vibrio mimicus, Vibrio parahaemolyticus, Vibrio alginolyticus, Vibrio fluvialis, Vibrio vulnificus, Vibrio vulnificus, Campylobacter jejuni, Staphylococcus aureus, Bacillus cereus and Escherichia coli, in which case the nucleic acid molecules may include one or more base sequences selected from among SEQ ID NOS: 43 to 70, SEQ ID NO: 134, SEQ ID NOS: 87 and 88, SEQ ID NOS: 97 and 98, SEQ ID NOS: 101 and 102, and SEQ ID NOS: 117 to 130, or one or more base sequences substantially identical thereto. When the bacteria is Listeria monocytogenes, the nuclei acid molecules may include a base sequence selected from among SEQ ID NOS: 135 and 136, or SEQ ID NOS: 137 and 138, or a base sequence substantially identical thereto. When the bacteria is Salmonella enteritidis, the nucleic acid molecules may include a base sequence selected from among SEQ ID NOS: 139 and 140, or a base sequence substantially identical thereto. When the bacteria is Yersinia enterocolitica, the nucleic acid molecules may include a base sequence selected from among SEQ ID NOS: 143 and 144, or a base sequence substantially identical thereto.

Alternatively, the bacteria may be one or more species selected from among the genera Neisseria, Chlamydia and Mycoplasma. These bacteria arc causative organisms for sexually transmitted diseases. If the bacteria is Neisseria gonorrhoae, Neisseria meningitides, Chlamydia trachomatis or Mycoplasma genitarium, the nucleic acid molecules may include one or more base sequences selected from among SEQ ID NOS: 71 to 86, SEQ ID NOS: 105 and 106, and SEQ ID NOS: 109 to 114, or one or more base sequences substantially identical thereto.

The present invention may also provide a process for identifying species of bacteria, in which one or more species of bacteria selected from among bacteria of the genera Streptococcus, Staphylococcus, Enterococcus, Peptostreptococcus, Klebsiella, Escherichia, Enterobacter, Legionella, Neisseria, Bacillus, Helicobacter, Corynebacterium, Pseudomonas, Burkholderia, Haemophilus, Bacteroides, Enterococcus, Nocardia, Prevotella, Neisseria, Moraxella, Flavobacterium, Clostridium, Treponema, Sphingobacterium, Leptospira, Campylobacter, Listeria, Salmonella and Yersinia are subject bacteria which are the subject of identification, and the process includes the step of (a) acquiring the base sequence of at least a portion of the DnaJ gene in a test sample which may contain the nucleic acid of the subject bacteria.

This identification step preferably includes the step of (b) identifying a species of bacteria within the test sample by comparing the base sequence of at least the portion of the DnaJ gene acquired from the test sample with a base sequence of the DnaJ gene of the bacteria to be identified.

The invention may also provide a bacterial identification program for identifying bacteria, in which one or more species of bacteria selected from among bacteria of the genera Streptococcus, Staphylococcus, Enterococcus, Peptostreptococcus, Klebsiella, Escherichia, Enterobacter, Legionella, Neisseria, Bacillus, Helicobacter, Corynebacterium, Pseudomonas, Burkholderia, Haemophilus, Bacteroides, Enterococcus, Nocardia, Prevotella, Neisseria, Moraxella, Flavobacterium, Clostridium, Treponema, Sphingobacterium, Leptospira, and Campylobacter are subject species of bacteria being identification subjects. The program entails running, on one or more computer, the step of identifying the species of bacteria within a test sample which may contain the nucleic acid of the bacteria to be identified by comparing the base sequence of at least a portion of the DnaJ gene acquired from the test sample with the base sequence of the DnaJ gene of the bacteria to be identified.

The present invention may also provide nucleic acid molecules for detecting and identifying bacteria, in which one or more species of bacterial, selected from the genera Streptococcus, Staphylococcus, Enterococcus, Peptostreptococcus, Klebsiella, Escherichia, Enterobacter, Legionella, Neisseria, Bacillus, Helicobacter, Corynebacterium, Pseudomonas, Burkholderia, Haemophilus, Bacteroides, Enterococcus, Nocardia, Prevotella, Neisseria, Moraxella, Flavobacterium, Clostridium, Treponema, Sphingobacterium, Leptospira, Campylobacter, Listeria, Salmonella and Yersinia are subjects of detection and identification, and the nucleic acid molecules hybridize with at least a portion of the DnaJ gene of the bacteria to be identified. The nucleic acid molecules may be primers for gene amplification, or may be probes. Alternatively, one or more type of nucleic acid molecule selected from the above may be rendered into the form of solid phase body in which the nucleic acid molecules have been immobilized in a solid phase, or into the form of a bacterial identification and detection kit which includes one or more type of nucleic acid molecule selected from such nucleic acid molecules.

The inventors have also discovered that by separating the functions of the primers into two, microorganisms can be detected rapidly and to a high sensitivity. That is, the inventors have found that by combining a first primer set, having both a sequence that specifically anneals to a target nucleic acid and a tag sequence which does not anneal to the target nucleic acid, with a second primer set having a tag sequence that is substantially the same as the tag sequence of the first primer set, the target sequences can be detected to an acceptable sensitivity even in cases where a plurality of primer sets directed at a plurality of types of targets are made to function at the same time. Moreover, the inventors have also found that, in order to create a mixture of primers (also referred to below as a "cocktail primer") capable of detecting a plurality of types of microorganisms, species-specific primers can easily be designed by targeting the DnaJ gene of each species of microorganism, thus amplifying the target at a good amplification efficiency in what is substantially a single PCR reaction cycle, and enabling highly sensitive detection. This discovery ultimately led to the present invention. The invention may provide the following means.

The invention may additionally provide a method for amplifying at least one type of target nucleic acid, which method includes a step which carries out a polymerase chain reaction using a first primer set having both a tag sequence and a base sequence specific to the target nucleic acid and at least one type of a second primer set which has a tag sequence that is substantially the same as the tag sequence of the first primer set.

The polymerase chain reaction step may be a step which carries out an amplification of the target nucleic acid with the first primer set and also an amplification of the amplified product thereby obtained with the second primer set. Also, the polymerase chain reaction step may be a step which carries out substantially a single PCR reaction cycle.

The polymerase chain reaction step may be a step which carries out a single PCR reaction cycle wherein the first primer set has a primer concentration of at least 0.005 µM and at most 0.1 µM, the second primer set has a primer concentration which is at least 10 times and at most 50 times the primer concentration of the first primer set, and the amplified product obtained with the second primer set is a nucleic acid of up to 250 bases.

The invention may further provide a multiplex PCR-based method for diagnosing, the pathogenic microorganisms of two or more species selected from the genera Staphylococcus, Streptococcus, Serratia, Klebsiella, Escherichia, Mycobacterium, Legionella, Vibrio, Bacillus, Neisseria, Campylobacter, Chlamydia Chlamydophila, Mycoplasma, Listeria, Salmonella and Yersinia. The method includes a polymerase chain reaction step of carrying out, on a test sample which may contain nucleic acid from the pathogenic microorganisms, a polymerase chain reaction using at least one type of a first primer set having a tag sequence and a base sequence which selectively anneals to a target nucleic acid (preferably on the DnaJ gene) possessed by the pathogenic microorganism, and using at least one type of a second primer set having a tag sequence which is substantially the same as the tag sequence of the first primer set; and a step of detecting an amplified product containing the target nucleic acid. In this method, it is preferable for the polymerase chain reaction step to carry out a single PCR reaction cycle.

The invention may provide also a kit containing primer sets for amplifying, by multiplex PCR, a target nucleic acid of at least one type of microorganism, which kit includes a first primer set having a tag sequence and a base sequence which selectively anneals to a target nucleic acid (preferably on the DnaJ gene) possessed by the pathogenic microorganism, and a second primer set which has substantially the same tag sequence as the tag sequence of the first primer set.

### Brief Description of the Drawings

FIG. 1A compares 16S rDNA sequences. Only 12 bases (1.2%) differ between the two bacterial species.
FIG. 1B shows the polymorphism of DnaJ sequences. Here, 183 bases (20.6%) differ between the two bacterial species.
FIG. 2 shows DnaJ primer sequences for S. aureus.
FIG. 3 is a diagram showing the results of amplification with a single primer (primer solution B) and multiple primers (primer solution A).
FIG. 4 is a diagram showing the results obtained for DNA amplified in a LightCycler (Roche) capillary tube by monitoring the rise in CYBR Green fluorescence intensity.
FIG. 5 shows the results obtained from the confirmation of PCR amplified products by agarose electrophoresis.
FIG. 6 compares, by means of electrophoresis, the detection sensitivities achieved with gene amplification.
FIG. 7 is a diagram showing the results obtained for DNA amplified in a LightCycler (Roche) capillary tube by monitoring the rise in CYBR Green fluorescence intensity.
FIG. 8 shows 55 species and 3 subspecies type strains of genus Mycobacterium for which the DnaJ gene was sequenced.
FIG. 9 compares DnaJ gene sequences and RpoB gene sequences.
FIG. 10 compares the base sequences of various genes.
FIG. 11 compares the degrees of similarity among 16S rDNA gene base sequences and among DnaJ gene base sequences.
FIG. 12 shows identification results obtained for various strains using the DnaJ gene, and identification results obtained using biochemical properties.
FIG. 13 shows the identification results obtained based on the base sequences of the DnaJ gene for 16 clinically isolated strains (the KPM strains in the diagram), which were collectively called the M. avium - M. intracellulare group (MAC group).
FIG. 14 is a diagram showing the relationships between the first primer set and the second primer set used in the present method of amplification method, and the target nucleic acid.
FIG. 15 shows primer sequences designed for Legionella pneumophila GTC 1279 DNA as the target nucleic acid.
FIG. 16 is a graph showing the amplification monitoring results in the PCR step carried out in Example 8.
FIG. 17 is a graph showing the amplification monitoring results in the PCR step carried out in Example 9.
FIG. 18 shows primer sequences for detecting bacterial pathogens in diarrhea.
FIG. 19 shows primer sequences for detecting viral pathogens in diarrhea.
FIG. 20 shows the probe sequences immobilized on a silicon substrate in Example 10.
FIG. 21 shows the fluorescence when the PCR product in Example 10 was hybridized with probes on a silicon substrate.
Fig. 22 shows primer sequences for the detection of STD pathogenic microorganisms.
Fig. 23 shows the probe sequences used for detecting amplified products in the PCR product in Example 11.
FIG. 24 shows the fluorescence intensities of the hybridization products obtained in Example 11.
FIG. 25 shows the primers making up the four cocktail primers prepared in Example 12.
FIG. 26 shows the CTm values obtained as a result of carrying out PCR in Example 12.

### Best Mode for Carrying Out the Invention

The present method of detection disclosed herein is able to detect specific species of microorganisms by detecting the presence or absence of hybridization when the above-described test sample is brought into contact with nucleic acid molecules which hybridize with at least a portion of the DnaJ gene in the species to be detected. The DnaJ gene, while having a low degree of similarity between microbial species, is preserved at or above a certain level between the strains of the same species. For this reason, species-specific base sequences can easily be extracted, and specific species can easily be detected using such base sequences. In particular, because the degree of similarity between species is low, even among related species, specific species can be detected to a good sensitivity. Also, the low degree of similarity between species enables specific primers and the like to be designed. As a result, it is possible to detect specific species even using a mixture of a plurality of primer sets. Hence, a plurality of species can be detected using a single primer mixture containing multiple types of primer sets. The DnaJ gene, inasmuch as it is a housekeeping gene indispensable for sustaining life and is possessed by all strains of microorganisms, is advantageous for the detection and identification of microbial species.

Moreover, the present method of detection is able to detect the microbial species to be identified by, for example, given the low degree of similarity of the DnaJ gene between bacterial species, analyzing the DnaJ gene, such as through determination of the base sequence of the DnaJ gene in the bacteria to be identified or hybridized with a given probe, and then comparing the results with the results of analyses on the base sequences of the DnaJ gene in existing related species.

Embodiments of the invention are described in detail below with respect to, for example, the present methods for detecting and identifying bacteria, and nucleic acid molecules used in such methods.

### Method of Detecting Bacteria

### Bacteria to be Detected

The present process for detecting bacteria is targeted at the detection of one or more species of bacteria selected from among bacteria of the genera Staphylococcus, Streptococcus, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia. In the bacterial species belonging to these genera, because the base sequence of the DnaJ gene has a lower degree of similarity than the base sequence of the 16S rDNA gene, finding species-specific base sequences that are preserved between strains of the same species is easy, making the DnaJ gene suitable for use in detecting bacterial species. For example, with respect to the DnaJ gene, in bacteria of the genus Staphylococcus the similarity in base sequence between species averages 97.4% for the 16S rDNA gene and averages 77.6% for the DnaJ gene; in bacteria of the genus Streptococcus, the similarity in base sequence between species averages 95.8% for the 16S rDNA gene and averages 76.4% for the DnaJ gene; in bacteria of the genus Mycobacterium, the similarity in base sequence between species averages 93% for the 16S rDNA gene and averages 83.7% for the DnaJ gene; and in bacteria of the genus Legionella, the similarity in base sequence between species averages 96.1 % for the 16S rDNA gene and averages 82.5% for the DnaJ gene. In bacteria of the genus Vibrio, the similarity in base sequence between species averages 97.4% for the 16S rDNA gene and averages 82% for the DnaJ gene; in bacteria of the genus Aeromonas, the similarity in base sequence between species averages 98.8% for the 16S rDNA gene and averages 89.8% for the DnaJ gene; and in bacteria of the family Enterobacteriaceae, the similarity in base sequence between species averages 97.1 % for the 16S rDNA gene and averages 87.6% for the DnaJ gene.

Of these, the bacterial species to be assayed are preferably species of bacteria belonging to the genera Klebsiella, Escherichia, Vibrio, Bacillus, Neisseria, Campylobacter, Chlamydia, Chlamydophila, Mycoplasma, Listeria, Salmonella and Yersinia. This is because the detection and identification of bacterial species of these genera by genetic engineering techniques has hitherto been difficult, and the use of the DnaJ gene will now make detection and identification by genetic engineering possible.

Even within the Enterobacteriaceae family, which has highly similar 16S rDNA genes, if one examines the differences between species within a single genus, among the four species of the genus Klebsiella, the 16S rDNA gene differs by only 1.4%, whereas the DnaJ gene differs by 7.8%. In the genus Serratia, the 16S DNA gene differs by 2.3%, whereas the DnaJ gene differs by 5.5%. In the genus Salmonella, the 16S rDNA gene differs by 1.4%, whereas the DnaJ gene differs by 6.3%, indicating greater polymorphism. Thus, by using the DnaJ gene, it is possible to suitably detect bacteria and identify the species.

In the present method of detection, by using a mixture of nucleic acid molecules which respectively detect a plurality of microbial species, two or more species can be targeted for detection. The reason is that, because the base sequence of the DnaJ gene has a low degree of similarity between genera and between microbial species but has sequence sites with a high degree of similarity between strains, it is possible to design nucleic acid molecules which hybridize with the DnaJ gene without mutual interference.

### Test Sample

The present method of detection may include the step of (a) contacting a test sample which is expected to contain nucleic acids of the bacteria to be detected with a nucleic acid molecule which hybridizes with at least a portion of the DnaJ gene of the species of bacteria that is the subject of detection. Here, the test sample is not subject to any particular limitation, provided it may contain nucleic acids of the species of bacteria to be detected. Illustrative examples of the test sample include blood, serum, cells or tissue collected from animals, including man, excrements such as feces and urine, phlegm, well water and food products. The test sample may be a nucleic acid extraction sample obtained by extracting nucleic acid from such collected material, or may be a sample containing an amplified product of the DnaJ gene or a portion thereof (which includes the region to be detected).

### Nucleic Acid Molecules

Nucleic acid molecules that can be used in the method of the invention may include one or more bacterial species-specific base sequence extracted by aligning the base sequences of the DnaJ gene of strains belonging to the same species. Such nucleic acid molecules are species-specific and enable strains belonging to the same bacterial species to be reliably detected. Software such as DNASIS pro ver. (Hitachi Software), Beacon designer ver. 5.1 (Molecular Beacon) and Clustal W (free software) may be used for such alignment and characteristic sequence extraction.

The nucleic acid molecules may include one extracted species-specific base sequence, or may include two or more. Alternatively, the nucleic acid molecules may include a base sequence which is substantially identical to a species-specific base sequence that is extracted in the aforementioned procedures. Here, "substantially identical" is exemplified by base sequences which are based on the above-described base sequence and have been modified within a range capable of maintaining the specificity of hybridization with the DnaJ gene. Illustrative examples include the above base sequence, the complementary base sequence, and base sequences in which one or more base in the above base sequence has been modified in one or more form selected from among substitution, insertion, deletion and addition. Such modifications may be carried out so as to have the base sequence correspond with the base sequence of the DnaJ gene for a specific strain.

The nucleic acid molecules are, specifically, primers and probes which have such specific base sequences or include such base sequences. The nucleic acid molecules are not subject to any particular limitation, insofar as they are capable of hybridization based on the base sequence. Although they are typically DNA, the nucleic acid molecules may take the form of DNA, RNA, DNA-RNA chimeras, artificial nucleic acids containing modified bases, or peptide nucleic acids (PNA). Such nucleic acid molecules may be modified with various fluorescent dyes, fluorescent dye quenchers or the like.

Next, examples of preferred nucleic acid molecules are given for each bacterial species. When the bacteria is Streptococcus pyogenes, exemplary nucleic acid molecules include those containing one or more base sequences selected from among SEQ ID NOS: 1 to 4 (primers) and SEQ ID NOS: 89 and 90 (probes), or one or more base sequences substantially identical thereto. When the bacterial species is Streptococcus agalactiae, exemplary nucleic acid molecules include those containing one or more base sequences selected from among SEQ ID NOS: 5 to 8 (primers) and SEQ ID NOS: 91 and 92 (probes), or one or more base sequences substantially identical thereto. When the bacteria is Streptococcus pneumoniae, exemplary nucleic acid molecules include those containing one or more base sequences selected from among SEQ ID NOS: 9 to 12 (primers) and SEQ ID NOS: 93 and 94 (probes), or one or more base sequences substantially identical thereto. When the bacteria is Staphylococcus aureus, exemplary nucleic acid molecules include those containing one or more base sequences selected from among SEQ ID NOS: 13 to 16 (primers) and SEQ ID NOS: 97 and 98 (probes), or one or more base sequences substantially identical thereto. When the bacteria is Klebsiella pneumoniae, exemplary nucleic acid molecules include those containing one or more base sequences selected from among SEQ ID NOS: 17 to 20 (primers) and SEQ ID NOS: 109 to 110 (primers), or one or more base sequences substantially identical thereto. When the bacteria is Escherichia coli, exemplary nucleic acid molecules include those containing one or more base sequences selected from among SEQ ID NOS: 21 to 24 (primers) and SEQ ID NOS: 101 and 102 (probes), or one or more base sequences substantially identical thereto. When the bacterial species is Mycobacterium tuberculosis, exemplary nucleic acid molecules include those containing one or more base sequences selected from among SEQ ID NOS: 25 to 28 (primers) and SEQ ID NOS: 103 and 104 (probes), or one or more base sequences substantially identical thereto. When the bacteria are Mycobacterium spp. (exclusive of the M. tuberculosi group), exemplary nucleic acid molecules include those containing one or more base sequences selected from among SEQ ID NOS: 29 and 30 (primers) and SEQ ID NOS: 131 to 133 (primers), or one or more base sequences substantially identical thereto. When the bacteria is Legionella pneumophila, exemplary nucleic acid molecules include those containing one or more base sequences selected from among SEQ ID NOS: 35 to 38 (primers) and SEQ ID NOS: 115 and 116 (probes), or one or more base sequences substantially identical thereto. When the bacteria is Vibrio cholerae, exemplary nucleic acid molecules include those containing one or more base sequences selected from among SEQ ID NOS: 43 to 46 (primers) and SEQ ID NOS: 127 and 128 (probes), or one or more base sequences substantially identical thereto. When the bacteria is Vibrio mimicus, exemplary nucleic acid molecules include those containing one or more base sequences selected from among SEQ ID NO: 43, SEQ ID NOS: 47 to 49 (primers) and SEQ ID NOS: 125 and 126 (probes), or one or more base sequences substantially identical thereto. When the bacteria is Vibrio parahaemolyticus, exemplary nucleic acid molecules include those containing one or more base sequences selected from among SEQ ID NO: 43, SEQ ID NOS: 50 to 52 (primers), SEQ ID NOS: 123 and 124 (probes), or one or more base sequences substantially identical thereto. When the bacteria is Vibrio vulnificus, exemplary nucleic acid molecules include those containing one or more base sequences selected from among SEQ ID NO: 43, SEQ ID NOS: 53 to 55 (primers) and SEQ ID NOS: 119 and 120 (probes), or one or more base sequences substantially identical thereto. When the bacteria is Vibrio fluvialis, exemplary nucleic acid molecules include those containing one or more base sequences selected from among SEQ ID NO: 43, SEQ ID NO: 134, SEQ ID NOS: 56 and 57 (primers), and SEQ ID NOS: 117 and 118 (probes), or one or more base sequences substantially identical thereto. When the bacteria is Vibrio alginolyticus, exemplary nucleic acid molecules include those containing one or more base sequences selected from among SEQ ID NO: 43, SEQ ID NOS: 58 to 60 (primers), and SEQ ID NOS: 121 and 122 (probes), or one or more base sequences substantially identical thereto. When the bacteria is Bacillus cereus, exemplary nucleic acid molecules include those containing one or more base sequences selected from among SEQ ID NOS: 61 to 66 (primers) and SEQ ID NOS: 87 and 88 (probes), or one or more base sequences substantially identical thereto. When the bacteria is Campylobacter jejuni, exemplary nucleic acid molecules include those containing one or more base sequences selected from among SEQ ID NOS: 67 to 70 (primers) and SEQ ID NOS: 129 and 130 (primers), or one or more base sequences substantially identical thereto. When the bacteria is Neisseria meningitidis, exemplary nucleic acid molecules include those containing one or more base sequences selected from among SEQ ID NOS: 83 to 86 (primers) and SEQ ID NOS: 111 and 112 (probes), or one or more base sequences substantially identical thereto. When the bacteria is Neisseria gonorrohae, exemplary nucleic acid molecules include those containing one or more base sequences selected from among SEQ ID NOS: 79 to 82 (primers) and SEQ ID NOS: 113 and 114 (probes), or one or more base sequences substantially identical thereto. When the bacteria is Chlamydia trachomatis, exemplary nucleic acid molecules include those containing one or more base sequences selected from among SEQ ID NOS: 75 to 78 (primers) and SEQ ID NOS: 109 and 110 (probes), or one or more base sequences substantially identical thereto. When the bacteria is Chlamydophila pneumoniae, exemplary nucleic acid molecules include those containing one or more base sequences selected from among SEQ ID NOS: 39 to 42 (primers) and SEQ ID NOS: 107 and 108 (probes), or one or more base sequences substantially identical thereto. When the bacteria is Mycoplasma genitarium, exemplary nucleic acid molecules include those containing one or more base sequences selected from among SEQ ID NOS: 71 to 74 (primers) and SEQ ID NOS: 105 and 106 (probes), or one or more base sequences substantially identical thereto. When the bacteria is Mycoplasma pneumoniae, exemplary nucleic acid molecules include those containing one or more base sequences selected from among SEQ ID NOS: 31 to 34 (primers) and SEQ ID NOS: 95 and 96 (probes), or one or more base sequences substantially identical thereto.

In detecting the causative organisms for pneumonia or pharyngitis, the organism to be detected may be one or more bacteria from the genera Streptococcus, Staphylococcus, Klebsiella, Escherichia, Mycobacterium, Legionella, Chlamydia, Chlamydophila, Neisseria and Mycoplasma. Here, when the bacteria is selected from among Streptococcus pyogenes, Streptococcus agalactiae, Streptococcus pneumoniae, Staphylococcus aureus, Klebsiella pneumoniae, Escherichia coli, Mycobacterium tuberculosis, Legionella pneumophila, Chlamydia trachomatis, Chlamydophila pneumoniae, Neisseria gonorrhoae, Neisseria meningitides and Mycoplasma pneumoniae, exemplary nucleic acid molecules include those containing one or more base sequences selected from among SEQ ID NOS: 1 to 42, SEQ ID NOS: 131 to 133 and SEQ ID NOS: 89 to 116, or nucleic acid molecules containing one or more base sequences substantially identical thereto. Of these, it is preferable to combine primer sets for the respective DnaJ genes of two or more bacterial species.

In detecting the causative organisms for diarrhea or food poisoning, the organism to be detected may be one or more bacteria from the genera Vibrio, Campylobacter, Staphylococcus, Bacillus, Escherichia, Listeria, Salmonella and Yersinia. Here, when the bacteria is one selected from among Vibrio cholerae, Vibrio mimicus, Vibrio parahaemolyticus, Vibrio alginolyticus, Vibrio fluvialis, Vibrio vulnificus, Vibrio vulnificus, Campylobacter jejuni, Staphylococcus aureus, Bacillus cereus and Escherichia coli, exemplary nucleic acid molecules include those containing one or more base sequences selected from among SEQ ID NOS: 43 to 70, SEQ ID NO: 134, SEQ ID NOS: 87 and 88; SEQ ID NOS: 97 and 98, SEQ ID NOS: 101 and 102, and SEQ ID NOS: 117 to 130, or nucleic acid molecules containing one or more base sequences substantially identical thereto. When the bacteria is Listeria monocytogenes, exemplary nucleic acid molecules include those containing the base sequences in SEQ ID NOS: 135 and 136 or SEQ ID NOS: 137 and 138, or nucleic acid molecules containing base sequences substantially identical thereto. When the bacteria is Salmonella enteritidis, exemplary nucleic acid molecules include those containing the base sequences in SEQ ID NOS: 139 and 140, or nucleic acid molecules containing base sequences substantially identical thereto. When the bacteria is Yersinia enterocolitica, exemplary nucleic acid molecules include those containing the base sequences in SEQ ID NOS: 143 and 144, or nucleic acid molecules containing base sequences substantially identical thereto. Of these, it is preferable to combine primer sets for the respective DnaJ genes of two or three or more bacterial species.

In detecting the causative organisms for sexually transmitted diseases, the organism to be detected may be one or more bacteria from the genera Neisseria, Chlamydia and Mycoplasma. In such cases, when the bacteria is Neisseria gonorrhoae, Neisseria meningitides, Chlamydia trachomatis or Mycoplasma genitarium, exemplary nucleic acid molecules include those containing one or more base sequences selected from among SEQ ID NOS: 71 to 86, SEQ ID NOS: 105 and 106, and SEQ ID NOS: 109 to 114, or nucleic acid molecules containing one or more base sequences substantially identical thereto. Of these, it is preferable to combine primer sets for the respective DnaJ genes of two or more bacterial species.

Base sequences preferable for use in the nucleic acid molecules are listed below together with the names of the bacteria to be detected thereby. The nucleic acid molecules used as primers are given below as basically one set composed of a forward primer and a reverse primer, in this order.

### Preferred Nucleic Acid Molecule Base Sequences for Use as Primers

Primers of Causative Organisms for Airway Infections
Streptococcus pyogenes
SEQ ID NO: 01: 5-ATAAGGATGTTCAAGAAGCTTACG
SEQ ID NO: 02: 5-CACCAAAGCCGCCTTGAG
SEQ ID NO: 03: 5-TTTTGAAGAGGCTGTATTTGG
SEQ ID NO: 04: 5-GAGCGGTACCAGGTTTTG
Streptococcus agalactiae
SEQ ID NO: 05: 5-GCTTATGAAACCTTGAGTGATAC
SEQ ID NO: 06: 5-AAACCGCCACCATCGAAG
SEQ ID NO: 07: 5-GGAGATGACCTACAATATCG
SEQ ID NO: 08: 5-GGCTAGTACCTGGCTTAG
Streptococcus pneumoniae
SEQ ID NO: 09: 5-GCCTATGAGACTTTGAGTGAC
SEQ ID NO: 10: 5-ACCAGCTCCACCAAAACC
SEQ ID NO: 11: 5-GCAATCCAAACGCTCCTC
SEQ ID NO: 12: 5-TGTACGACAGCCAGCTTC
Staphylococcus aureus
SEQ ID NO: 13: 5-GGACAAGGATTCAATGGCTCT
SEQ ID NO: 14: 5-TTGCGGTGCATTTGGATCTCT
SEQ ID NO: 15: 5-GAAGCGGTATTTGGTACAAC
SEQ ID NO: 16: 5-GCCATTACAGTAACTACAAGTC
Klebsiella pneumoniae
SEQ ID NO: 17: 5-CCCTGTCGGTTAAAATTCC
SEQ ID NO: 18: 5-CTCAAAGATAGCGTGCTG
SEQ ID NO: 19: 5-GCGTAGAAAAGACCAAAACC
SEQ ID NO: 20: 5-CAGGTTCAGGTGAAGCAG
Escherichia coli
SEQ ID NO: 21: 5-TGTTGAGCGCAGCAAAAC
SEQ ID NO: 22: 5-CAAGACGGATGCGGTCTC
SEQ ID NO: 23: 5-CTCGAAGAAGCTGTACGTG
SEQ ID NO: 24: 5-CTGTGTACCTGGTTTTGC
Mycobacterium tuberculosis
SEQ ID NO: 25: 5-GTTCGACAGCGGCTTTGG
SEQ ID NO: 26: 5-GAACAAGTCGTTGAGGTTGAAC
SEQ ID NO: 27: 5-AAGACTTCTACCAGGAGCTG
SEQ ID NO: 28: 5-ACTTCCGATAGGCACGTTT
Mycobacterium spp. (aside from M. tuberculosis)
SEQ ID NO: 29: 5-CGIGARTGGGTYGARAARG
SEQ ID NO: 30: 5-GGIGAYYTITTCGGIGGIYT
SEQ ID NO: 131: 5-GTGARTGGGTCGARAARGACT
SEQ ID NO: 132: 5-CGIGTYTCGTCGTAYTCCTT
SEQ ID NO: 133: 5-CAGCGRTTACCYGCCCA
Mycoplasma pneumoniae
SEQ ID NO: 31: 5-AGCTTGCTATGCAGTACC
SEQ ID NO: 32: 5-CTCTTTAAACTTCTCTTCGTTTTG
SEQ ID NO: 33: 5-TTGAGATGACTAATGGTTGTACTC
SEQ ID NO: 34: 5-CCCTTCAGCCCCAAAACC
Legionella pneumophila
SEQ ID NO: 35: 5-ACTGTTTGTGAGGGATCG
SEQ ID NO: 36: 5-AAAACCTTGTTGAATTCTGAC
SEQ ID NO: 37: 5-TTGAAGAAGCGGCTATAGG
SEQ ID NO: 38: 5-CTCACAAACAGTACAAGTACC
Chlamydophila pneumoniae
SEQ ID NO: 39: 5-TCTCAGTGATCCTCAGAA
SEQ ID NO: 40: 5-CCAAAGGCTCCCATGAAAG
SEQ ID NO: 41: 5-TTTGGAATGCGCTCAGATC
SEQ ID NO: 42: 5-GCTTCTTCAAAAGTCAAATTAATATG

Primers of Organisms which Cause Diarrhea and Food Poisoning Vibrio cholerae
SEQ ID NO: 43: 5-CAGGTTTGYTGCACGGCGAAGA
SEQ ID NO: 44: 5-AGCAGCTTATGACCAATAGCC
SEQ ID NO: 45: 5-AAACACGTCACCGAAAATATC
SEQ ID NO: 46: 5-TTATGACCAATACGGCCATG
Vibrio mimicus
SEQ ID NO: 43: 5-CAGGTTTGYTGCACGGCGAAGA
SEQ ID NO: 47: 5-YCTTGAAGAAGCGGTTCGTGCA
SEQ ID NO: 48: 5-ATATCGCCAAAGTCAG
SEQ ID NO: 49: 5-GCATCGGTCAGAATTTCATAC
Vibrio parahaemolyticus
SEQ ID NO: 43: 5-CAGGTTTGYTGCACGGCGAAGA
SEQ ID NO: 50: 5-TGCGAAGAAAGGCTCATCAGAG
SEQ ID NO: 51: 5-CCAAAGATGTCGCCGAAG
SEQ ID NO: 52: 5-CGAAGTTCTAACCGACTCTC
Vibrio vulnificus
SEQ ID NO: 43: 5-CAGGTTTGYTGCACGGCGAAGA
SEQ ID NO: 53: 5-GTACGAAATTCTGACCGATCAA
SEQ ID NO: 54: 5-CCACCGCCTTGTTCAAAAG
SEQ ID NO: 55: 5-ACGAAATTCTGACCGATCC
Vibrio fluvialis
SEQ ID NO: 43: 5-CAGGTTTGYTGCACGGCGAAGA
SEQ ID NO: 134: 5-GTACGAAATTCTGACCGATCAA
SEQ ID NO: 56: 5-CAATCTCTTTCGAGCAACCAC
SEQ ID NO: 57: 5-CAACGTGGTGCGGATCTG
Vibrio alginolyticus
SEQ ID NO: 43: 5-CAGGTTTGYTGCACGGCGAAGA
SEQ ID NO: 58: 5-GATCGAAGTRCCRACACTMGGA
SEQ ID NO: 59: 5-CCGAATACATCGCCAAAG
SEQ ID NO: 60: 5-ATGCTGCTTTTGAACAAGG
Bacillus cereus
SEQ ID NO: 61: 5-GATCAATTTGGTCATGCTGGT
SEQ ID NO: 62: 5-GAAGCCACCACCGAAGTC
SEQ ID NO: 63: 5-AAAGCGTATCGTCGTTTGG
SEQ ID NO: 64: 5-ATCATCACTTAACACTTCATATGC
SEQ ID NO: 65: 5-CGCAGTACGATCAATTTGG
SEQ ID NO: 66: 5-CAAAGAAAGAACTAAATATATCTTC
Campylobacter jejuni
SEQ ID NO: 67: 5-TACTTATAAATGCTCTTGTAAAAC
SEQ ID NO: 68: 5-CTTTGGACAAGTTTGAAG
SEQ ID NO: 69: 5-TAGACTTTACTTATAAATGCTCTTG
SEQ ID NO: 70: 5-GACACTTTGGACAAGTTTG

Primers of Causative Organisms for Sexually Transmitted Diseases Mycoplasma genitarium
SEQ ID NO: 71: 5-GAAGGGTTAAATGCTTCTGG
SEQ ID NO: 72: 5-CACCATCCATTCCAAAGG
SEQ ID NO: 73: 5-AAGGGATTATTATGAAGTTCTAGGG
SEQ ID NO: 74: 5-CATTTTCTGCTTTATGACGATC
Chlamydophila trachomatis
SEQ ID NO: 75: 5-GGGATTTCCTTCTTGATCC
SEQ ID NO: 76: 5-CATGGGGATGATTTAGTTTTAG
SEQ ID NO: 77: 5-AAAGAAACCGCCTCCATTA
SEQ ID NO: 78: 5-GCATGGGGAATATGGAAGAC
Neisseria gonorrhoae
SEQ ID NO: 79: 5-GGCGGGGAGTTAGAAGTG
SEQ ID NO: 80: 5-GACACCCTTACCTTTCACG
SEQ ID NO: 81: 5-CGGGCAAACATATTAAAGAACC
SEQ ID NO: 82: 5-CGGGAATATTGACTTCCAC
Neisseria meningitidis
SEQ ID NO: 83: 5-TTGGAAGTGCCGACCTTG
SEQ ID NO: 84: 5-GATTTGACACCCTTACCCTTCG
SEQ ID NO: 85: 5-GGGCAAACACATTAAAGAACC
SEQ ID NO: 86: 5-GCGGGAATATTGACTTCCA

Base Sequences of Nucleic Acid Molecules Preferred for Use as Probes Bacillus cereus
SEQ ID NO: 87: TTACCATCCAGACGTAAGTAAAGAAGAA
SEQ ID NO: 88: TTAGCCACCGCCGAAGTCTCCTCC
Streptococcus pyogenes
SEQ ID NO: 89: TTCGCCGTATTGATCATAAGCAGCA
SEQ ID NO: 90: TTCCTGAGCCTAAACAAGTGCCG
Streptococcus agalactiae
SEQ ID NO: 91: TTAAAACCGCCATTTGCCCCAG
SEQ ID NO: 92: TCTTCCTGAACAAGTATGACATGATGAC
Streptococcus pneumoniae
SEQ ID NO: 93: TTCGTGCTGCCTATGACCAGTATG
SEQ ID NO: 94: TCGATGATACTTAACTTCCTTCTCAGT
Mycoplasma pneumoniae
SEQ ID NO: 95: TACAGTCTCACCCTCACCCTTATG
SEQ ID NO: 96: TTCACAGGCACTACAGGTCACC
Staphylococcus aureus
SEQ ID NO: 97: TTCAATCCGTAAAGATGTAACATGCG
SEQ ID NO: 98: TTCAATCCGTAAAGATGTAACATGCGAA
Klebsiella pneumoniae
SEQ ID NO: 99: TTCGTACAGATCGCCTGCCG
SEQ ID NO: 100: CGCGCCGGCAGGCGATCTGTACGT
Escherichia coli
SEQ ID NO: 101: AAGAGATCCGCATTCCGACTCTGGAAG
SEQ ID NO: 102: TTAAGAGATCCGCATTCCGACT
Mycobacterium tuberculosis
SEQ ID NO: 103: TCGGGGTCGGTGGAGACGGCG
SEQ ID NO: 104: TTCTCCTCTGATGCCAGTCCTGAAGA
Mycoplasma genitarium
SEQ ID NO: 105: TAGCAGGGTTTAATCCTTTTGACATCT
SEQ NO: 106: TAAACGCTAGTTCTCAAGACATAAA
Chlamydophila pneumoniae
SEQ ID NO: 107: TTCGCAAGOCATCTTCCATGTTCC
SEQ ID NO: 108: TTTCTTACTGGCTCCTTGACGA
Chlamydia trachomatis
SEQ ID NO: 109: TTAGCCGCATCAACAAATCCAATAGG
SEQ ID NO: 110: TTACCGAAATCGCCGCCAAA
Neisseria meningitidis
SEQ ID NO: 111: TTCTTGACCGCCTTATTCCGCC
SEQ ID NO: 112: TGCCGTGGCGCGGGGCGGAATA
Neisseria gonorrhoeae
SEQ ID NO: 113: TTCTTGACCGCCTTATTCCGCC
SEQ ID NO: 114: TGCCGTGGCGTGGGGCGGAATA
Legionella pneumophila
SEQ ID NO: 115: TAAGAAGTTGAAATTACCGTTCCAAGAC
SEQ ID NO: 116: AAAGAAGTTGAAATTACCGTTCC
Vibrio fluvialis
SEQ ID NO: 117: TTCCAGCGTCAGTTCCATGTTG
SEQ ID NO: 118: TCGAGCAGCCGCGTACCGCTTCT
Vibrio vulnificus
SEQ ID NO: 119: TCCATATTGATCGTAAGCCGCT
SEQ ID NO: 120: TCGAGACACCACGAACGGCCTCT
Vibrio alginolyticus
SEQ ID NO: 121: TTAATCAGCACCGCCGCCAC
SEQ ID NO: 122: TCGTTACACCACGAACCGCTTCT
Vibrio parahaemolyticus
SEQ ID NO: 123: TTCAGCACCGCCTCCACCAA
SEQ ID NO: 124: TAGTTACACCACGAACGGCTTCT
Vibrio mimicus
SEQ ID NO: 125: TTCGAAGCCGCCGCCACCAA
SEQ ID NO: 126: TCGAACATCCACGAACCGCTTCT
Vibrio cholerac
SEQ ID NO: 127: TTAATCAGCACCGCCACCGC
SEQ ID NO: 128: TCGAGCAGCCGCGAACCGCTTCT
Campylobacter jejuni
SEQ ID NO: 129: TGTAATGGAACAGGGGCTA
SEQ ID NO: 130: TCCATCTTTAGCCCCTGTTCCAT

To bring a test sample and nucleic acid molecules into contact, the DnaJ gene of the organism to be detected which may be present in the test sample and the nucleic acid molecules should be placed in a hybridizable state. For example, both the DnaJ gene and the nucleic acid molecules may be made present in a liquid phase, or one of these may be bound to a solid phase and the other may be included in a liquid phase which comes into contact with the solid phase. As subsequently described, the step of achieving such contact may be carried out as part of a base sequencing process which is based either on a gene amplification technique such as PCR, RT-PCR, transcription mediated amplification (TMA), a branched DNA (bDNA) method, real-time PCR or the ligase chain reaction (LCR), or on nucleic acid hybridization such as the Southern blotting method, a microarray method, a microbead method or nucleic acid (DNA) chromatography. The technique employed in this step may also be selected as appropriately depending on whether, for example, the nucleic acid molecules to be used are designed as primers or are designed as probes.

The present method may include the step of detecting whether the nucleic acid molecules have hybridized with nucleic acids within the test sample. Detecting whether such hybridization has taken place may be carried out by, for example, (I) directly detecting the presence or absence of specific hybridization between the nucleic acid molecules and nucleic acids in the test sample, (2) the presence or absence of gene amplification reactions based on specific hybridization between the nucleic acid molecules and nucleic acids in the test sample, and (3) detecting the presence or absence of specific base sequences in the products of a gene amplification reaction.

In the case of (1) above, hybridization can be detected by, for example, adding a labeling compound which generates a detectable signal on the nucleic acid molecules and/or the nucleic acids extracted from the test sample, or adding a signal-forming compound which generates a change that generates a signal by intercalation or the like via hybridization. To efficiently detect the hybridization products, it is advantageous to bond the nucleic acid molecules, or nucleic acids in the test sample, to a solid phase. Such detection may be carried out as a base sequencing technique based on the hybridization of nucleic acid, such as the Southern blotting method, a microarray method, a microbead method (flow cytometry), or nucleic acid (DNA) chromatography. The nucleic acid molecules may take the form of probes.

In the case of (2) above, it is possible, for example, by having the nucleic acid molecules function as primers and inducing the elongation of nucleic acid molecules that have specifically hybridized with the DnaJ gene as the template, to detect by electrophoresis or the like whether a given amplified product has been obtained. Such gene amplification and detection may be carried out by a gene amplification method or amplified product detection method, such as PCR, RT-PCR, transcription-mediated amplification (TMA), the branched DNA (bDNA) method, real-time PCR or a ligase chain reaction (LCR) method. A method which uses PCR is described in detail later in this specification.

The approach in (3) above may be carried out by combining the above (2) and (1). For example, it is possible to obtain a gene amplification product by using, as the nucleic acid molecules, primers which are specific to a fixed range of genera or species, and also to detect the presence or absence of hybridization with respect to the resulting gene amplification product by using, as the nucleic acid molecules, probes which are specific to specific species.

### Method of Identifying Bacteria

The present method of identifying bacteria may include the step of acquiring at least a portion of the base sequence of the DnaJ gene in a test sample, such as a clinical sample, which may contain the bacteria to be identified or nucleic acids thereof. By acquiring at least a portion of the base sequence of the DnaJ gene, it is possible to identify the species of the bacteria to be identified. Moreover, a phylogenetic tree thereof can be prepared. Species of bacteria which are preferable as the target of detection in the present method of detecting bacteria include bacteria of the genera Streptococcus, Staphylococcus, Enterococcus, Peptostreptococcus, Klebsiella, Escherichia Enterobacter, Legionella, Neisseria, Bacillus, Helicobacter, Corynebacterium, Pseudomonas, Burkholderia, Haemophilus, Bacteroides, Enterococcus, Nocardia, Prevotella, Neisseria, Moraxella, Flavobacterium, Clostridium, Treponema, Sphingobacterium, Leptospira, Campylobacter, Listeria, Salmonella and Yersinia. Because the DnaJ gene is a gene that is polymorphic in these bacteria, it is useful for identifying bacterial species thereamong. When acquiring the base sequence of the DnaJ gene, the base sequence of a PCR amplified product obtained using the above-mentioned nucleic acid molecules as primers or the base sequence of a hybridization product obtained using the above-mentioned nucleic acid molecules as probes may be acquired.

The base sequence may be acquired by, for at least a portion of a gene obtained by the PCR method or the like, a base sequencing method carried out with a sequencer that employs a known technique, or a method (involving the use of, for example, a microarray) that entails hybridization with a probe having a given base sequence.

When identifying the species based on at least a portion of the base sequence of the DnaJ gene acquired from the test sample, it is preferable to compare the acquired base sequence with a previously acquired base sequence of the identification subject DnaJ gene of the bacteria. That is, when identifying bacterial species, it is advantageous to first prepare a database of base sequences for the complete or partial DnaJ gene of each species of bacteria. Preparing such a database beforehand enables the bacterial species to be rapidly identified. It is also advantageous to prepare a database containing information on the base sequences of the DnaJ gene for the various strains within the bacterial species. With such a database, species-specific base sequences common to the strains can be easily extracted. By using the base sequences extracted in this way, bacterial species can be easily and accurately identified. At the same time, this is also advantageous for creating a phylogenetic tree.

In particular, when identifying bacterial species by means of the DnaJ gene, it is useful to first carry out the step of acquiring the base sequence of the 16S rDNA gene of the test sample. This is because, although the degree of similarity with related species is high in the base sequence of the 16S rDNA gene, the degree of similarity in the DnaJ gene is low. For example, by first determining a candidate species having the highest degree of similarity in the 16S rDNA gene (the number of such species may be one or may be two or more), then determining the base sequence of the DnaJ gene in the candidate species and the base sequence of the DnaJ gene of an isolated strain or the like in the test sample, the species of the isolated strain can be easily and rapidly determined. In cases where identification is possible with the 16S rDNA gene, there is no need for identification with the DnaJ gene. While no particular limitation is imposed on the degree of similarity with the 16S rDNA gene of existing species when using such a method, when the base sequences of the 16S rDNA gene in an isolated strain and in an existing bacterial species are at least 98% similar, it is preferable to compare the base sequences of the DnaJ gene in this existing strain of bacteria and in the isolated strain.

As is the case when acquiring the base sequence of the DnaJ gene, a suitable, known technique may be used to acquire the base sequence of the 16S rDNA gene. Also, by comparing the base sequence of at least a portion of the 16S rDNA gene in the test sample with the base sequence of the 16S rDNA gene in an existing bacterial species, the degree of similarity between these base sequences can be advantageously obtained. It is desirable to prepare beforehand a database of the base sequences of the 16S rDNA gene for the various bacterial species or strains.

When analyzing the base sequence of the DnaJ gene and the like in the test sample, although the sampling source for the bacteria to be identified is not subject to any particular limitation, as already noted, use may be made of test samples which include, for example, clinical samples. Moreover, as with the method of detecting bacteria already described, a nucleic acid extract of a bacteria or an amplified product of a given region of a bacterial gene may be used as the test sample.

In addition, given the above, the present invention also may provide a bacterial identification program for obtaining at least a portion of the base sequence of the DnaJ gene. Letting a bacteria similar to that in the above method of identification be the bacteria to be identified, the program entails running, on one or more computer, the step of identifying the species of bacteria within a test sample which may contain the nucleic acid of the bacteria to be identified by comparing the base sequence of at least a portion of the DnaJ gene acquired from the test sample with the base sequence of the DnaJ gene in the bacteria to be identified. With this program, the sequence of the DnaJ gene obtained from the test sample can be used to identify the species of bacteria in the test sample by retrieving from a DnaJ gene sequence a database sequence which matches the base sequence. In such a program, the above step may be preceded by the step of comparing at least a portion of the base sequence of the 16S rDNA gene obtained from the above-described test sample with the base sequence of the 16S rDNA gene in the bacteria to be identified, determining candidate species having 16S rDNA gene base sequences of the highest degree of similarity with the acquired base sequence, then comparing the base sequences of the DnaJ gene in these candidate species with the base sequence of the DnaJ gene from the test sample.

The form of the program is not a matter of particular concern. The program may be in a form downloaded over the Internet, or may be held in a suitable storage medium. Alternatively, the program may be stored in the ROM, etc. of a computer or other suitable piece of equipment. In addition, according to the present invention, an apparatus which stores such a program in an executable manner may be provided. The controller of such an apparatus may have a CPU capable of running such a program, a memory device such as a ROM or hard drive which stores the program, and a suitable sequence input means. In addition, a storage means such as a hard drive within the apparatus may be equipped with a DnaJ gene sequence database. In an environment where connection to the Internet is possible, the required processing may be carried out by accessing a server where such a sequence database has been stored. Also, the sequence comparison step, i.e., the step in which the degree of similarity between sequences is determined and alignment is carried out, may use all or portions of such software as DNASIS pro ver. (Hitachi Software), Beacon designer ver. 5.1 (Molecular Beacon) and Clustal W (free software).

### Nucleic Acid Molecules, and Bacteria Detection and Identification Kit Containing Nucleic Acid Molecules

The nucleic acid molecules of the invention may include the various types of nucleic acid molecules already described. Alternatively, such nucleic acid molecules may be prepared as a detection and identification kit for detecting or identifying bacteria containing one or more of the various above-mentioned nucleic acid molecules. The nucleic acid molecules selected for inclusion in such a detection and identification kit may be nuclear acid molecules which function as probes and/or primers. The combination of nucleic acid molecules in a detection and identification kit is not subject to any particular limitation, although it is preferable for the kit to be one in which nucleic acid molecules capable of detecting or identifying preferably at least three, more preferably at least four, and even more preferably at least five species of bacteria, are combined. An example of a preferred combination of the target bacterial species is one which includes two species of the genus Chlamydophila, one species of the genus Chlamydia, one species of the genus Coxiella, one species of the genus Haemophilus, four species of the genus Legionella, two species of the genus Streptococcus, three species of the genus Mycobacterium, one species of the genus Pseudomonas, one species of the genus Mycoplasma, one species of the genus Staphylococcus, one species of the genus Bordetella, one species of the genus Corynebacterium, one species of the genus Bordetella, one species of the genus Klebsiella, one species of the genus Serratia and one species of the genus Escherichia. A kit of such nucleic acid molecules is preferable for detecting and identifying causative organisms for pneumonia and pharyngitis. Another example of a preferred combination is one which includes one species of the genus Chlamydia, two species of the genus Neisseria, two species of the genus Mycoplasma, two species of the genus Ureaplasma, one species of the genus Candida, one species of the genus Treponema, one species of the genus Trichomonasa and two species of genus Haemophilus. A kit of such nucleic acid molecules is preferable for detecting and identifying the causative organisms for sexually transmitted diseases and urinary tract infections. An additional example of a preferred combination is one which includes two species of the genus Yersinia, four species of the genus Escherichia-Shigella, one species of the genus Campylobacter, one species of the genus Salmonella, four species of the genus Vibrio, three species of the genus Aeromonas, one species of the genus Plesiomonas, one species of the genus Giardia, one species of the genus Entamoeba and four species of the genus Clostridium. Such nucleic acid molecule kits are preferable for detecting and identifying the causative organisms of food poisoning and diarrhea.

The above preferred combinations may be used together particularly with a process in which the bacterial species are identified by means of an amplified product obtained by amplifying nucleic acid in the test sample by PCR or the like with 16S rDNA as the target thereof. For example, a primer cocktail (a composition) containing primer sets capable of amplifying the 16S rDNA gene may be utilized. First, identification of the bacterial species by means of 16S rDNA is attempted; if such identification is impossible, then identification with DnaJ may be attempted. It is possible in this way to detect or identify a plurality of bacterial species all at once, or to carry out the detection or identification of the bacterial species efficiently.

Such detection and identification kits may be prepared simply as a kit of one or more type of nucleic acid molecule, e.g., in a form which contains the nucleic acid molecule as a primer and is adapted for gene amplification; or may be prepared as a kit in which one or more nucleic acid molecule as a probe may be immobilized on a solid phase such as beads or a substrate.

### Immobilized Body

The immobilized body of the invention may be obtained by immobilizing one or more type of the above-described nucleic acid molecule on a solid phase such as beads or a substrate. The nucleic acid molecule immobilized on the solid phase is distinguished by positional information on the sequence thereof within a substrate or the like, or by the color thereof within beads. The nucleic acid molecule which has been immobilized on the solid phase may have bonded thereto a compound which emits a signal upon hybridization. Also, the nucleic acid molecule may be immobilized on the surface of a solid phase so as to enable a primer extension reaction. The form of the solid phase is not subject to any particular limitation. Nor is the material thereof subject to any particular limitation. For example, use may be made of glass, plastic or ceramic.

Also, the immobilizing body of the invention may be a nucleic acid chromatographic apparatus obtained by immobilizing the nucleic acid molecule as a probe on a chromatographic substrate such as a filter.

The present invention also relates to an amplification method for amplifying or detecting at least one type of target nucleic acid, and the use of such a method. These inventions are useful for amplifying and detecting nonspecific target nucleic acids, in addition to which they are useful in the detection and identification of bacteria using the base sequence of the DnaJ gene, in which the above-mentioned DnaJ gene or a portion thereof serves as the target nucleic acid. That is, by using a bacterial species-specific primer based on the base sequence of the DnaJ gene, it is possible to further enhance the usefulness of the present invention in the detection or identification of one or more species (preferably two or more species) of bacteria.

The target nucleic acid amplification method of the invention is a method of amplifying at least one type of target nucleic acid. This method includes the step of carrying out a polymerase chain reaction using at least one type of a first primer set having a tag sequence and a base sequence specific to the target nucleic acid, and using at least one type of a second primer set having a tag sequence which is substantially the same as the tag sequence of the first primer set.

With the present method of amplifying a target nucleic acid, a target nucleic acid is amplified by the first primer set, and the target nucleic acid sequence-containing amplified product obtained with the first primer set is amplified by the second primer set. That is, even when the efficiency of target nucleic acid amplification with the first primer set is low, amplification with the second primer set enables amplified product containing the amount of target nucleic acid required for detection to be easily obtained. Therefore, even when a plurality of types of the first primer set have low concentrations, by compensating for the low efficiency of amplification with the first primer set, one or more target nucleic acid can be amplified with the first primer set. Also, because the target sequence is amplified primarily by the second primer set, the amplification efficiency can easily be made uniform.

It is possible to have the above polymerase chain reaction step be a step which simultaneously carries out both amplification of the target nucleic acid using the above first primer set, and also of the amplified product obtained with the first primer set using the second primer set. Such a polymerase chain reaction step enables the target nucleic acid to be easily detected.

In the present invention, the first primer set may be designed so as to have a base sequence specific to a target nucleic acid on the DnaJ gene of a microorganism. By using the DnaJ gene, even when microorganisms of different genera and of different species in the same genus are present, the microorganisms can easily be detected at the species level. The inventors have shown that the DnaJ gene has base sequences which have a low degree of similarity between species but are preserved within the same species. Hence, the DnaJ gene is beneficial for detecting pathogenic microorganisms having differing levels of toxicity or malignancy depending on the species. Moreover, by using the DnaJ gene, the amplified product can easily be designed to a length of 250 bases or less, at which competition by the amplified product can be effectively avoided.

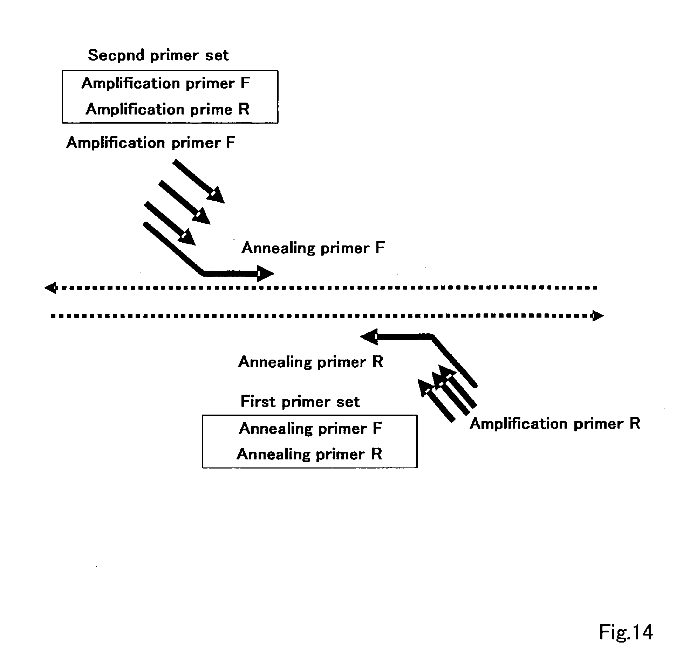

The present method of amplifying a target nucleic acid and the present kit for such amplification are described below in detail for the present embodiment while referring as appropriate to the diagrams. FIG. 14 shows the relationships between the first primer set and the second primer set used in the method of amplification herein, and the target nucleic acid.

### Polymerase Chain Reaction

In the present invention, "polymerase chain reaction (PCR)" refers to a reaction which amplifies a specific DNA sequence by means of an enzyme reaction within a test tube. PCR methods that exist include the RT-PCR method, the real-time PCR method, and diverse variants thereof, as well as combinations of these with other analytic techniques. As used in the present invention, the term "PCR" may encompass all of these.

### Target Nucleic Acid

In the present invention, the term 'target nucleic acid' refers to a nucleic acid which is all or part of the base sequence to be amplified by the polymerase chain reaction. The target nucleic acid is not subject to any particular limitation, provided amplification by the polymerase chain reaction is possible. Examples include the DNA or RNA which makes up the genome; a medium of genetic information for an organism, as well as mRNA, cDNA and cRNA. The target nucleic acid may be single-stranded or double-stranded.

The target nucleic acid may be extracted from an organism or a portion of tissue thereof, and may take any of various forms, such as amplified product obtained by a PCR reaction on mRNA, cDNA or a genome obtained by expression analysis. The form is not subject to any particular limitation. Nor is the organism or tissue serving as the source thereof subject to any particular limitation. In addition to animals, including both humans and nonhuman mammals, microorganisms such as bacteria, true fungi and viruses may be used as the source. From the standpoint of both preventing and treating infections and also hygiene, the source organism for the target nucleic acid is preferably a microorganism, more preferably the microorganism to be detected or identified for the sake of hygiene or treatment, and even more preferably a pathogenic microorganism.

Examples of microorganisms from which the target nucleic acid originates include bacteria of the genera Staphylococcus, Streptococcus, Serratia, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia. The microorganisms of one or more species of bacteria are selected from those belonging to these genera. The species belonging to these genera have preferred target nucleic acids on the DnaJ gene. The reason is that, in the microorganisms belonging to these genera, the base sequence of the DnaJ gene has a lower degree of similarity between species than does the base sequence of the 16S rDNA gene, thus making it easier to find species-specific base sequences which are preserved between strains within the same species.

Of the above, species of bacteria of the genera Staphylococcus, Streptococcus, Mycobacterium, Legionella and Vibrio are preferred. The reason is that the bacteria belonging to these genera have a low degree of similarity in the DnaJ gene between genera and between species in the same genus, making them suitable for detecting the bacterial species by means of the DnaJ gene. For example, with respect to the DnaJ gene, in bacteria of the genus Staphylococcus the average degree of similarity between species is 97.4% in the 16S rDNA gene and 77.6% in the DnaJ gene; in bacteria of the genus Streptococcus, the average degree of similarity between species is 95.8% in the 16S rDNA gene and 76.4% in the DnaJ gene; in bacteria of the genus Mycobacterium, the average degree of similarity between species is 93% in the 16S rDNA gene and 83.7% in the DnaJ gene; in bacteria of the genus Legionella, the average degree of similarity between species is 96.1 % in the 16S rDNA gene and 82.5% in the DnaJ gene; and in bacteria of the genus Vibrio, the average degree of similarity between species is 97.4% in the 16S rDNA gene and 82% in the DnaJ gene.

The target nucleic acid is furnished as a test sample, such as blood, serum, cells or tissue collected from animals, including man, excrements such as feces and urine, phlegm, well water and food products. The test sample may be a nucleic acid extraction sample obtained by extracting nucleic acid from such collected material, or may be a sample containing an amplified product of the DnaJ gene or a portion thereof (which includes the region to be detected).

### First Primer Set

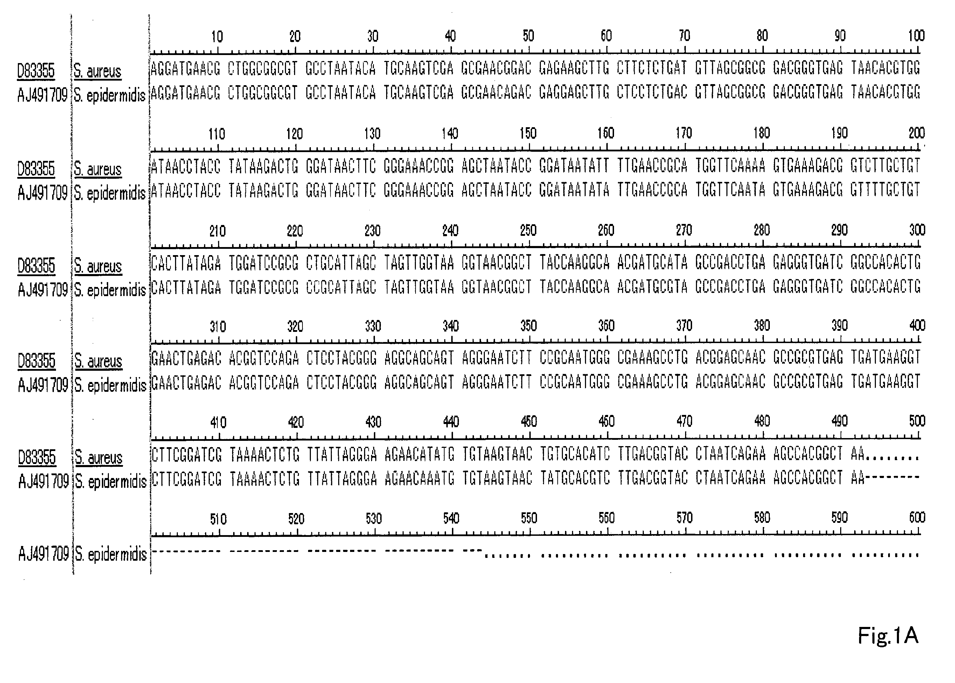

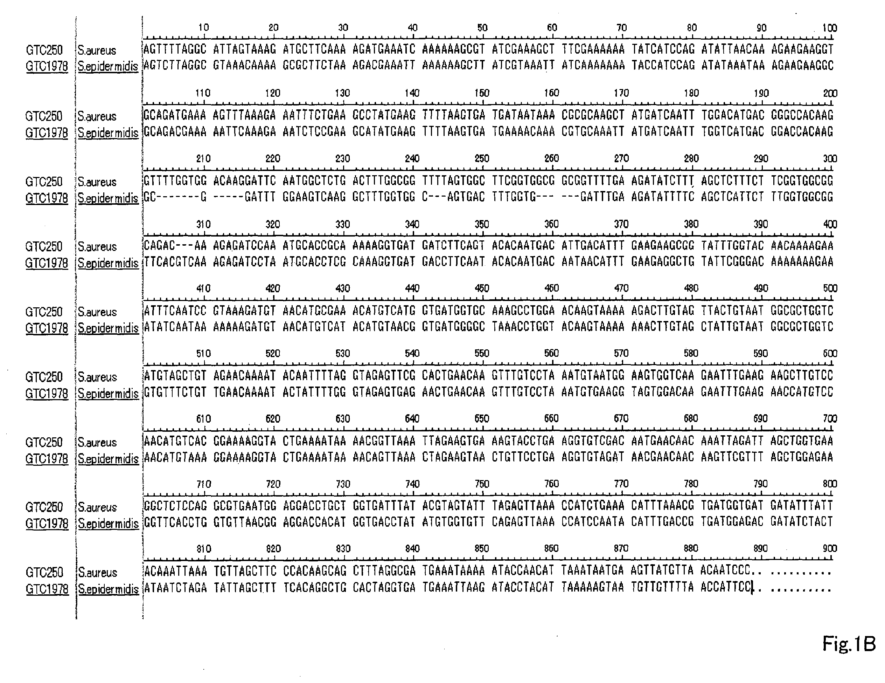

In the amplification method of the invention, a first primer set having both a tag sequence and a base sequence which selectively anneals to the target nucleic acid is used. The first primer set is composed of a forward primer and a reverse primer. As shown in FIG. 1, both the forward primer and the reverse primer have on the 5' side thereof a tag sequence, and have on the 3' side a sequence that anneals to the target nucleic acid.

The first primer set is selected in accordance with the type of target nucleic acid. Generally, a single first primer set is selected for a single target nucleic acid. However, cases in which a single first primer set is selected for two or more target nucleic acids are also possible.

It is preferable to select, as the base sequence which selectively anneals to the target nucleic acid (also called the "annealing sequence" below) and is included in each primer of the first primer set, a sequence having a high specificity which hybridizes and anneals only to the target nucleic acid. In cases where primers are designed for, as the target nucleic acid, the same gene, the annealing sequence may partially or completely match between different primer sets. The annealing sequence may be determined after taking into consideration the specificity to the target nucleic acid and also the avoidance of primer dimer formation and the reaction temperature in the PCR step. The reaction temperature Tm between the base sequence and the target nucleic acid is preferably at least 50°C but not above 70°C, and more preferably at least 55°C but not above 65°C.

When the amplification method is carried out in order to detect or identify microorganisms, a sequence specific for the microorganism to be detected or identified may be selected as this annealing sequence, although, as mentioned above, it is preferable to use a base sequence on the DnaJ gene as the target nucleic acid. When a base sequence on the same gene is used as the target nucleic acid for a plurality of microorganisms, a microorganism-specific base sequence can be selected by aligning the base sequences of the DnaJ gene for the plurality of microorganisms. Such an annealing sequence is species-specific, and can be reliably used to detect strains belonging to the species. Software such as DNASIS pro ver. (Hitachi Software), Beacon designer ver. 5.1 (Molecular Beacon) and Clustal W (free software) may be used for such alignment and characteristic sequence extraction.

The tag sequence may be selected from sequences which do not hybridize with the target nucleic acid which is to be amplified. The tag sequence preferably has a length of at least 12 bases but not more than 40 bases, and more preferably at least 18 bases but not more than 30 bases.

The forward primer and reverse primer which compose the first primer set may each have a linker sequence or the like between the tag sequence and the annealing sequence, although they are preferably composed only of two types of sequences. The forward primer and the reverse primer each have overall preferably at least 30 bases but not more than 80 bases, and more preferably at least 35 bases.

The amplified product obtained by amplification with such a first primer set preferably has at least 80 bases but not more than 280 bases. Within this range, the possibility that a common, preserved sequence is included in a gene of pathogenic microorganism or the like is reduced, increasing the sensitivity of detection. It is more preferable for the amplified product to have not more than 250 bases. At up to 250 bases, the possibility that a preserved sequence common to different bacterial species is included in the amplified region is reduced, enabling the amplification efficiency and sensitivity to be increased. It is even more preferable for the amplified product to have not more than 200 bases. At 200 bases or less, species-specific primers and probes are easy to design. Particularly in cases where the DnaJ gene of microorganism is used as the target nucleic acid, for the DnaJ gene structural reasons, at an amplified product length in excess of 200 bases, species-specific primers and probes are more difficult to design, whereas at 200 bases or less, species-specific primers are easier to design. Also, in the case of the DnaJ gene, for its gene structural reasons, even when the amplified product has this length, designing a specific probe is easier than doing so from the 16s rDNA. That is, by placing the target nucleic acid on the DnaJ gene, a first primer set can be designed so as to enable an amplified product of preferably up to 250 bases, and more preferably up to 200 bases, to be obtained in a plurality of microorganisms. In the present invention, because amplified product prepared using the first primer set can be obtained at a base length within such a fixed range, it is possible to make the PCR cycle for further amplifying this amplified product with the second primer set and the PCR reaction cycle using the first primer set one and the same cycle.

At least one type of the first primer set is used; in cases where the amplification method of the invention carries out an amplification step by multiplex PCR, a plurality of types of first primer sets may be used. It is preferable to use six or more types of first primer sets, and more preferable to use ten or more types of first primer sets. The use of 15 or more types of first primer sets is even more preferred. However, it is preferable for not more than 50 types of first primer sets to be used. When the target nucleic acid of a microorganism is amplified, it is preferable to use a sequence on the DnaJ gene as the target nucleic acid. Because using this gene enables primers to be designed which are species-specific and for which competition between amplified products does not readily arise, the target nucleic acids of a plurality of species of microorganisms can be easily detected by multiplex PCR.

### Second Primer Set

In the present amplification method, at least one type of second primer set having tag sequences that are each substantially the same as the tag sequences used in the first primer set may be used. The second primer set anneals via the tag sequences to the amplified product obtained by annealing of the first primer set to the target nucleic acid, thus enabling further amplification of the amplified product.

The second forward primer and the second reverse primer making up the second primer set each have tag sequences which are substantially the same as, respectively, the tag sequence for the first forward primer and the tag sequence for the first reverse primer. The second forward primer and the second reverse primer are preferably composed of tag sequences which are substantially the same as tag sequences for, respectively, the first forward primer and the first reverse primer.

Here, "tag sequences which are substantially the same as tag sequences" are exemplified by tag sequences which are the same as the tag sequence of the first forward primer, and base sequences that have been modified within a range where the specificity of hybridization between the first tag sequence and a complementary base sequence can be maintained. Illustrative examples include base sequences complementary to this base sequence, and base sequences obtained by modification, within this base sequence, in one or more form selected from among the substitution, insertion, deletion and addition of one or more bases. The use of tag sequences which are the same as the tag sequences which have been added respectively to the first primer set is preferred.

The second primer set is not used for a specific first primer set; rather, it is used correspondingly in accordance with the tag sequences which may be used in the first primer set. Therefore, in cases where two or more types of first primer sets employed in an amplification reaction use one tag sequence pair, there is one type of second primer set. Alternatively, in cases where two or more types of first primer sets use two or more tag sequence pairs, two or more types of second primer sets are used.

Such first primer set and second primer set are preferably used in such a way that the respective primer concentrations of the first primer set are low, and the respective primer concentrations of the second primer set are made higher than the respective primer concentrations of the first primer set. The reason is that, because the second primer set is used, the first primer set need only be able to produce amplified product to a degree capable of serving as a template for the second primer set.

The concentrations of the respective primers in the second primer set within the PCR reaction solution are preferably at least 10 times but not more than 50 times the concentrations of the respective primers in each of the first primer sets in the PCR reaction solution. Within these ranges, even when a plurality of types of first primer sets are used, it is possible to obtain a detection sensitivity which is comparable to that achieved when amplification is carried out with a single primer set. The ratio between the primer concentrations for the first primer set and the primer concentrations for the second primer set is preferably set in accordance with the number of types of first primer sets used. For example, when ten types of first primer sets are used, it is preferable for the respective primer concentrations of the second primer sets to be at least ten times the primer concentrations of the respective first primer sets. Similarly, when there are twenty types of first primer sets, it is preferable for the primer concentrations of the second primer sets to be at least twenty times higher.

The respective primer concentrations of the first primer sets are preferably at least 0.005 µM, but not more than 0.2 µM. Within this range, amplified product can be produced in a degree that is amplifiable by the second primer set. The primer concentration is more preferably not more than 0.1 µM. The primer concentrations of the second primer set are preferably at least 0.1 µM, and more preferably at least 0.25 µM.

The first primer set and the second primer set used in the present invention preferably have one of the following characteristics:
(a) the amplified product obtained with the above-described first primer set is a nucleic acid having not more than 250 bases;
(b) the concentration of primer from the first primer set at the time of use is at least 0.005 µM but not more than 0.2 µM (preferably not more than 0.1 µM); or
(c) the concentration of primer from the second primer set at the time of use is at least 10 times, but not more than 50 times, the concentration of primer from the first primer set at the time of use.
By satisfying any of these conditions, even when a plurality of types of first primer sets are used, the specificity is maintained and the target sequence can be amplified in the required amount. In addition, as described subsequently, an amplification step which uses a plurality of types of the first primer set can be carried out by a simple PCR step.

The nucleic acid molecules composing these first primer sets and second primer sets are not subject to any particular limitation, provided hybridization based on the base sequence is possible. Although the nucleic acid molecules are typically DNA, they may take the form of DNA, RNA, DNA-RNA chimeras, artificial nucleic acids containing modified bases, or peptide nucleic acids (PNA). Such nucleic acid molecules may be modified with various fluorescent dyes, fluorescent dye quenchers or the like.

### PCR Step

No particular limitations are imposed on the reaction conditions in the PCR step using the first primer set and the second primer set, so long as the target nucleic acid amplified product obtained with the first primer set can be amplified by the second primer set. The step in which the target nucleic acid is amplified with the first primer set and the step in which the amplified product of the first primer set is amplified with the second primer set may each be carried out by differing "denaturation-annealing-elongation" cycles. For example, the amplification step with the first primer set may be carried out at, in order, a temperature of at least 90°C but not above 97°C for 10 to 60 seconds, at a temperature of at least 55°C but not above 65°C for 10 to 60 seconds, and at a temperature of at least 70°C but not above 75°C for 1 to 30 seconds. The number of cycles is preferably at least one cycle but not more than several cycles. This is because, at a number of cycles exceeding ten, the degree of increase in the final amount of amplified product ceases to be large. The number of cycles is preferably one or two, and more preferably one. Also, the amplification step with the second primer set may be carried out at a temperature of at least 90°C but not above 97°C for 0.5 to 5 seconds, at a temperature of at least 55°C but not above 65°C for 0.5 to 5 seconds, and at a temperature of at least 70°C but not above 75°C for 1 to 20 seconds. The number of cycles is preferably at least 20 cycles but not more than 70 cycles, more preferably at least 3 0 cycles, and even more preferably at least 40 cycles but not more than 60 cycles.

The amplification step with the first primer set and the amplification step with the second primer set may be carried out in a single "denaturation-annealing-elongation" PCR cycle. In particular, when the length of the amplified product obtained with the first primer set is set to preferably 250 bases or less, and more preferably 200 bases or less, it is desirable for the conditions used in the PCR reaction cycle with the second primer set to be the same as the conditions used in the PCR cycle with the first primer set. In such a case, the first primer set and the second primer set may each be made to effectively function in a PCR cycle under the same conditions, thus enabling the amplified product to be efficiently obtained with the second primer set. This obviates the need to separately carry out a PCR cycle for the first primer set.

As already explained, by placing the target nucleic acid on the DnaJ gene, when the length of the amplified product obtained with a plurality of first primer sets for a plurality of target nucleic acids is 250 bases or less, and preferably 200 bases or less, carrying out a single PCR cycle is especially preferable in cases where multiplex PCR is to be carried out. By designing the amplified product of the first primer set to such a base length, competition at the time of amplification with the first primer set can be suppressed, in addition to which uniform amplification conditions can be applied to all the target nucleic acids, thus making it possible for any target nucleic acid to be detected at a comparable detection sensitivity. Moreover, having both the first primer set and the second primer set function within a single PCR cycle is advantageous as well for quantitative analysis, such as real-time PCR.

When the amplification step is carried out with the first primer set and the second primer set using a single PCR cycle, the step may be carried out at, in order, a temperature of at least 90°C but not above 97°C for 0.5 to 5 seconds, at a temperature of at least 55°C but not above 65°C for 0.5 to 5 seconds, and at a temperature of at least 70°C but not above 75°C for 1 to 20 seconds. The number of cycles is preferably at least 20 cycles but not more than 70 cycles, and more preferably at least 30 cycles but not more than 60 cycles.

In such a PCR step, it is preferable for the concentration of primer from the first primer set to be at least 0.005 µM but not more than 0.2 µM, and for the concentration of primer from the second primer set to be at least 10 times but not more than 50 times the concentration of primer from the first primer set. Within such a concentration range, the target nucleic acid can be efficiently amplified. Moreover, when the amplified product obtained with the first primer set is a nucleic acid having up to 250 bases, the possibility that it contains a region having a similar sequence decreases and competition during amplification does not arise, enabling the sensitivity to be increased.

In cases where the present invention is applied to the above-described method of detecting bacterial species, it is preferable to have the above step (a) be a step in which polymerase chain reactions are carried out using at least one type of first primer set having a tag sequence and a base sequence which selectively anneals to a portion of the DnaJ gene and at least one type of a second primer set having a tag sequence which is substantially the same as the tag sequence of the first primer set, and amplifications of at least a portion of the DnaJ gene by the first primer set as well as the amplified product obtained with the first primer set performed by the second primer set. In this way, even when there is a possibility that a plurality of species of bacteria are present, whether or not those species are present can be comprehensively detected for many species in a small number of experiments. Here, by having the PCR step be a step in which a single PCR reaction cycle is carried out, further simplification of the experiments is possible.

### Method of Diagnosing Pathogenic Microorganisms by Multiplex PCR

The present multiplex PCR-based method of diagnosing two or more species of pathogenic microorganisms selected from the genera Staphylococcus, Streptococcus, Serratia, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia includes the step of carrying out, on a test sample which may contain nucleic acid from the pathogenic microorganisms, a polymerase chain reaction using at least one type of a first primer set having a tag sequence and a base sequence which selectively anneals to a target nucleic acid on the DnaJ gene possessed by the pathogenic microorganisms, and using at least one type of a second primer set having a tag sequence which is substantially the same as the tag sequence of the first primer set; and the step of detecting an amplified product containing the target nucleic acid. The PCR step can be carried out similarly as described above. It is especially preferable for the PCR step to be a step which carries out a single PCR reaction cycle. In this way, the pathogenic microorganisms can be rapidly diagnosed. In the detection step, the method of detecting the amplified product is not subject to any particular limitation. For example, the amplified product may be detected by separation based on the molecular weight thereof using a technique such as electrophoresis. Alternatively, a labeled compound may be attached to the second primer, and the target sequence may be detected based on hybridization using the Southern blotting method, a microarray method, a microbead method (flow cytometry) or nucleic acid (DNA) chromatography.

The present method of diagnosis, by having the above-described PCR step, can detect the target nucleic acid specifically and to a high sensitivity even in cases where only a small amount of the target nucleic acid is present. Even when a plurality of types of primer sets are used and the target nucleic acids are detected by means of multiplex PCR, the target nucleic acids can be detected specifically, to a good sensitivity, and easily. Moreover, because the target nucleic acid is on the DnaJ gene, when a first primer set is designed by selecting as the annealing sequence a sequence which is preserved as much as possible on the Dna genes of different microorganisms, the first primer set uniformly amplifies the target nucleic acids on different DnaJ genes and the second primer set uniformly amplifies the resulting amplified products, thereby enabling the target nucleic acids to be quantitatively amplified, which in turn makes it possible to determine the amounts of those target nucleic acids, i.e., the amounts of the microorganisms. Such quantitative analysis may be applied also to other target nucleic acids. For example, it may be applied as well to target nucleic acids such as mRNA or cDNA which are the products of expression analysis.

### Kit Containing Primer Sets for Amplifying the Target Nucleic Acid of at Least One Type of Pathogenic Microorganism by Multiplex PCR

The present kit may include a first primer set having a tag sequence and a base sequence which selectively anneals to a target nucleic acid in a pathogenic microorganism, and a second primer set having a tag sequence which is substantially the same as the tag sequence of the first primer set. The embodiments of the first primer set and the second primer set in the present amplification method already described above are applicable also to the first primer set and second primer set in the present detection kit.

In addition, the present kit preferably has one of the following characteristics:
(a) the amplified product obtained with the above-described first primer set is a nucleic acid having not more than 250 bases;
(b) the concentration of primer from the first primer set at the time of use is at least 0.005 µM but not more than 0.2 µM (preferably not more than 0.1 µM); or
(c) the concentration of primer from the second primer set at the time of use is at least 10 times, but not more than 50 times, the concentration of primer from the first primer set at the time of use.

By satisfying any of these conditions, a small amount of the target nucleic acid can easily be amplified or detected. In addition, even when a plurality of primer sets is used at the same time, the target nucleic acid can be detected reliably and to a high sensitivity.

The present kit preferably has a target nucleic acid on the DnaJ gene. By having a target nucleic acid on the DnaJ gene, an amplified product can be designed so that, even when the amplified product obtained with the first primer set is a nucleic acid of 200 bases or less, it does not compete with other amplified products. In this detection kit, it is preferable for the target nucleic acid to be on the DnaJ genes of microorganisms. As already explained, when these microorganisms have a target nucleic acid on the DnaJ gene of one of the microorganisms shown in the table below, it is preferable to use the first forward primer and reverse primer shown in the same table.

**Table 1**

| | | |
|---|---|---|
| Mycobacterium tuberculosis DnaJ | Pneumonia primer F | 5-TAGCAGGATCCCTCTAAG-TTGTICGGTGGCTTGTTC |
| Mycobacterium tuberculosis DnaJ | Pneumonia primer R | 5-AATTCTAATACGACTCACTATAGGGAG-CCTCCACGAAATCCAACTC |
| Escherichia coli DnaJ | Pneumonia primer F | 5-TAGCAGGATCCCTCTAAG-TGTTGAGCGCAGCAAAAC |
| Escherichia coli DnaJ | Pneumonia primer R | 5-AATTCTAATACGACTCACTATAGGGAG-CAAGACGGATGCGGTCTC |
| Neisseria gonorrhoeae DnaJ | STD primer F | 5-TAGCAGGATCCCTCTAAGTTGGATTTGCATTGCGAACTG |
| Neisseria gonorrhoeae DnaJ | STD primer R | 5-AATTCTAATACGACTCACTATAGGGAG-CCAAGGTCGGCACTTCTAAC |
| Vibrio cholera DnaJ | Diarrhea primer F | 5-TAGCAGGATCCCTCTAAGCAGGTTTGYTGCACGGCGAAGA |
| Vibrio cholerae | Diarrhea primer R | 5-AATTCTAATACGACTCACTATAGGGAG- AGAAGCGGTTCGCGGCTGCTCG |
| Vibrio parahaemolyticus DnaJ | Diarrhea primer F | 5-TAGCAGGATCCCTCTAAGCAGGTTTGYTGCACGGCGAAGA |
| Vibrio parahaemolyticus | Diarrhea primer R | 5-AATTCTAATACGACTCACTATAGGGAG-GAAGCCGTTCGTGGTGTAACT |

The sequences of some DnaJ genes are known. In order to use the DnaJ gene as the target nucleic acid in the present invention, it was necessary to obtain, in the highly polymorphic DnaJ gene, sequences that are preserved at the species level and SNP polymorphism sites, but such sequences and sites were not known. The inventors thus sequenced the DnaJ gene in a large number of wild strains and identified regions that are species-specifically preserved and SNP sites, from which they were able to determine for the first time sequences for specific probes of the first primer set. They were also able to determine sequences for probes specific to the amplified product. The inventors learned in the above that, whereas the DnaJ gene has a polymorphism among strains of bacterial species belonging to the genus Mycobacterium of less than 1%, and of only from 1 to 3% among species of bacteria belonging to the family Enterobacteriaceae and among species of the genera Streptococcus and Staphylococcus, a difference of more than 5% exists between independent, related species.

The present kit may be a kit of primer sets for detecting one or more species of bacteria selected from the group of bacteria belonging to the genera Staphylococcus, Streptococcus, Serratia, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia. The primers are preferably designed so that portions of the sequences on the DnaJ genes in these organisms to be detected serve as the target nucleic acids. Primer sequences which amplify the above-described species-specific portions of the respective bacterial species belonging to these genera may be employed in primer design. The present kit preferably combines the primer sets for the respective DnaJ genes of two or more bacterial species among those to be detected.

The present kit may be prepared as a kit for detecting one or more species of bacteria selected from the above-described various causative organisms for pneumonia or pharyngitis. Alternatively, the present kit may be prepared as a kit for detecting one or more species of bacteria selected from the above-described various causative organisms for diarrhea and food poisoning. Alternately, the present kit may be prepared as a kit for detecting one or more species of bacteria selected from the above-described various causative organisms for sexually transmitted diseases. The present kit preferably uses portions of the DnaJ genes in these organisms to be detected as the target nucleic acids. Specific primer sequences for the respective causative organisms belonging to the above genera may be used to design the primers. In the present kit, it is preferable to combine primer sets for the respective DnaJ genes of two or more species of these organisms to be detected.

The present kit may be constituted so as to enable the target nucleic acids of preferably at least three, more preferably at least four, and even more preferably at least five, microorganisms to be amplified at the same time. For example, a preferred combination of target species in such a detection kit may include bacteria belonging to the genera Chlamydophila, Chlamydia, Coxiella, Haemophilus, Legionella Streptococcus, Mycobacterium, Pseudomonas, Mycoplasma, Staphylococcus, Bordetella, Corynebacterium, Bordetella, Klebsiella, Serratia and Escherichia. Such a kit is advantageous for detecting and identifying the causative organisms for pneumonia and pharyngitis. Alternatively, such a detection kit may include bacteria belonging to the genera Chlamydia, Neisseria, Mycoplasma, Ureaplasma, Candida, Treponema, Trichomonasa and Haemophilus. Such a kit is advantageous for detecting and identifying the causative organisms for sexually transmitted disease and urinary tract infections. Or such a detection kit may include bacteria belonging to the genera Yersinia, Escherichia-Shigella, Campylobacter, Salmonella, Vibrio, Aeromonas, Plesiomonas, Giardia, Entamoeba, Clostridium and Listeria. Such a kit is advantageous for detecting and identifying the causative organisms for food poisoning and diarrhea.

As stated above, according to the present invention, a process for detecting two or more species of bacteria selected from among bacteria of the genera Staphylococcus, Streptococcus, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia is provided; whereas the detection process includes the steps of (a) contacting a test sample which may contain nucleic acids of the bacteria to be detected with two or more types of nucleic acid molecules which hybridize with at least portions of the DnaJ gene in the two or more bacterial species to be detected; and (b) detecting whether the nucleic acid molecules have hybridized with nucleic acids within the test sample. Above Step (a) carries out a polymerase chain reaction using at least one type of a first primer set having a tag sequence and a base sequence which selectively anneals to a portion of the DnaJ gene and using at least one type of a second primer set having a tag sequence which is substantially the same as the tag sequence of the first primer set; and carries out, with the first primer set, amplification of at least a portion of the DnaJ gene and, with the second primer set, amplification of the amplified product obtained with the first primer set. The above polymerase chain reaction step may be carried out a single PCR reaction cycle.

Furthermore, a nucleic acid molecule kit for detecting and identifying two or more species of bacteria selected from among bacteria of the genera Streptococcus, Staphylococcus, Enterococcus, Peptostreptococcus, Klebsiella, Escherichia, Enterobacter, Legionella, Neisseria, Bacillus, Helicobacter, Corynebacterium, Pseudomonas, Burkholderia, Haemophilus, Bacteroides, Enterococcus, Nocardia, Prevotella, Neisseria, Moraxella, Flavobacterium, Clostridium, Treponema, Sphingobacterium, Leptospira, Campylobacter, Listeria, Salmonella and Yersinia may also be provided. The kit includes nucleic acid molecules which hybridize with at least portions of the DnaJ gene in the bacteria to be identified. In this kit, the nucleic acid molecules include at least one type of a first primer set having a tag sequence and a base sequence which selectively anneals to a portion of the DnaJ gene, and at least one type of a second primer set which has a tag sequence that is substantially the same as the tag sequence of the first primer set. Moreover, the invention also may provide a solid phase body in which two or more of these nucleic acid molecules making up such a nucleic acid molecule kit have been immobilized.

### Example 1

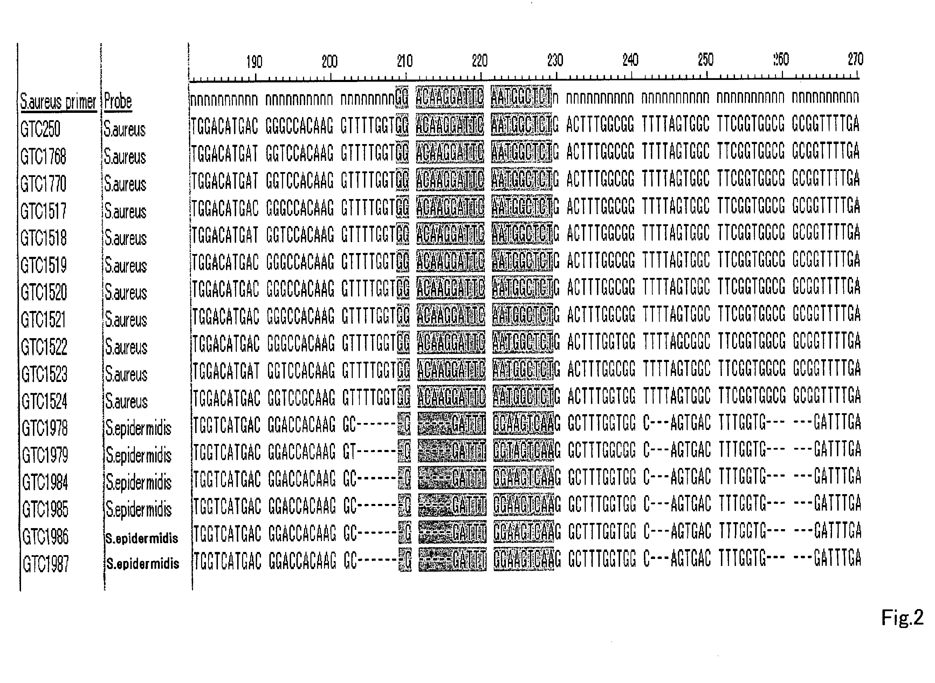

S. aureus of the genus Staphylococcus is a bacterial species serving as one cause in many of the food poisoning. In the 16S rDNA gene, its degree of similarity with the related species S. epidermidis is 99.5% (FIG. 1A), and as such, its species-specific primer design was impossible. However, in the DnaJ gene, the degree of similarity between the two species was found to be only 81.1% (FIG. 1B). This makes it possible to easily design a primer dedicated thereto. Moreover, the sequence of this primer is well preserved among 11 strains of S. aureus for which the sequences have been determined (FIG. 2). In the present example, primers were created for nine species of pathogens that cause food poisoning, based on the DnaJ sequences determined for these pathogens. The primer sequences for each of the pathogens were as follows.

Primers for Staphylococcus aureus
SEQ ID NO: 13: 5-GGACAAGGATTCAATGGCTCT
SEQ ID NO: 14: 5-TTGCGGTGCATTTGGATCTCT
Primers for Campylobacter jejuni
SEQ ID NO: 67: 5-TACTTATAAATGCTCTTGTAAAAC
SEQ ID NO: 68: 5-CTTTGGACAAGTTTGAAG
Primers for Vibrio cholerae
SEQ ID NO: 43: 5-CAGGTTTGYTGCACGGCGAAGA
SEQ ID NO: 44: 5-AGCAGCTTATGACCAATAGCC
Primers for Vibrio parahaemolyticus
SEQ ID NO: 43: 5-CAGGTTTGYTGCACGGCGAAGA
SEQ ID NO: 50: 5-TGCGAAGAAAGGCTCATCAGAG
Primers for Escherichia coli
SEQ ID NO: 21: 5-TGTTGAGCGCAGCAAAAC
SEQ ID NO: 22: 5-CAAGACGGATGCGGTCTC
Primers for Listeria monocytogenes
SEQ ID NO: 135: GTACAAGCTACATTAGGTGAC
SEQ ID NO: 136: GTTCGTTTGAAAATTCCAAGT
(or
SEQ ID NO: 137: GGACAACACCTGAAAAATGT
SEQ ID NO: 138: AATGGGGTATTTTGTTCCACGT)
Primers for Salmonella enteritidis
SEQ ID NO: 139: AAATCAAAAAGGCGTACAAG
SEQ ID NO: 140: GAGATTAAAGCAGCCTACGA
Primers for Yersinia spp.
SEQ ID NO: 141: CGTTCAGGTACAAGTCAAGG
SEQ ID NO: 142: GTGAGGTTCCTATTAACTTTGC
(or
Primers for Yersinia enterocolitica
SEQ ID NO: 143: TACGCGGTGTGACTAAAGAA
SEQ ID NO: 144: TATGTACCTGACCTGCACCA)
Primers for Clostridium perfringens (with 16S rDNA as the target nucleic acid)
SEQ ID NO: 145: GGTGCTTCAGATGATGAG
SEQ ID NO: 146: GGAGATAAGGAAGCGGAA

The above primers were mixed as indicated below to prepare a primer solution A containing all the primer sets for these nine species. In addition, the PCR reaction solution shown below was prepared.

| | |
|---|---|
| Primer solution A (containing 1 µM of each primer): | 2 µL |
| 2 x CYBER premix Hotstart Ex Taq (Takara): | 10 µL |
| S. aureus DNA (GTC 250 strain) | 5 ng |

The total volume of the above mixture was brought up to 20 µL with distilled water.

As a control, primer solution B was prepared using only the primers for S. aureus. In addition, the PCR reaction solution shown below was prepared.

| | |
|---|---|
| Primer solution B (containing 1 µM of each primer): | 2 µL |
| 2 x CYBER premix Hotstart Ex Taq (Takara): | 10 µL |
| S. aureus DNA (GTC 250 strain) | 5 ng |

The total volume of the above was brought up to 20 µL with distilled water.

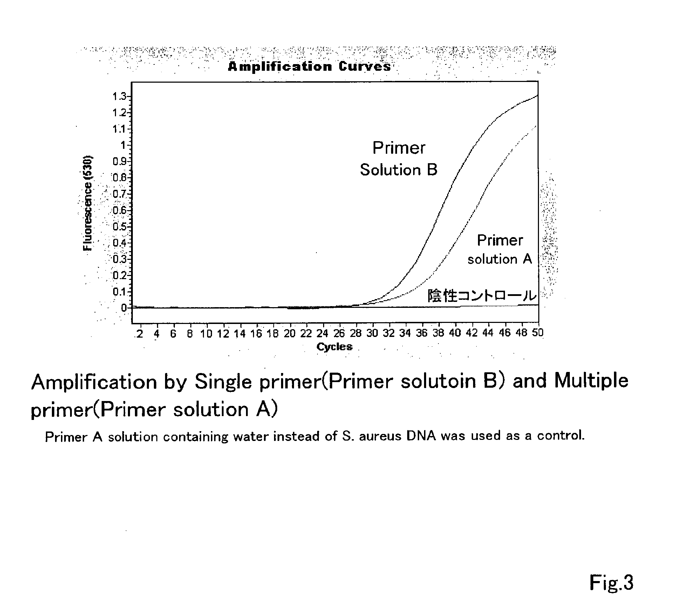

Each of the above PCR solutions were placed in a LightCycler (Roche) capillary tube along with reagents, and gene amplification (50 cycles, each consisting of 1 second at 95°C, 1 second at 55°C and 10 seconds at 72°C) was carried out with LightCycler. The results are shown in FIG. 3.

Amplification at substantially the same amplification efficiency was confirmed in the PCR product amplified in primer solution A and the PCR product amplified in primer solution B. That is, the amplification efficiency with the primer mixture containing nine types of primer sets and the amplification efficiency with the solitary primer set were at comparable levels, demonstrating that the respective sets of primer are able to amplify specific sequences of the DnaJ gene in the intended organisms without mutual interference.

### Example 2

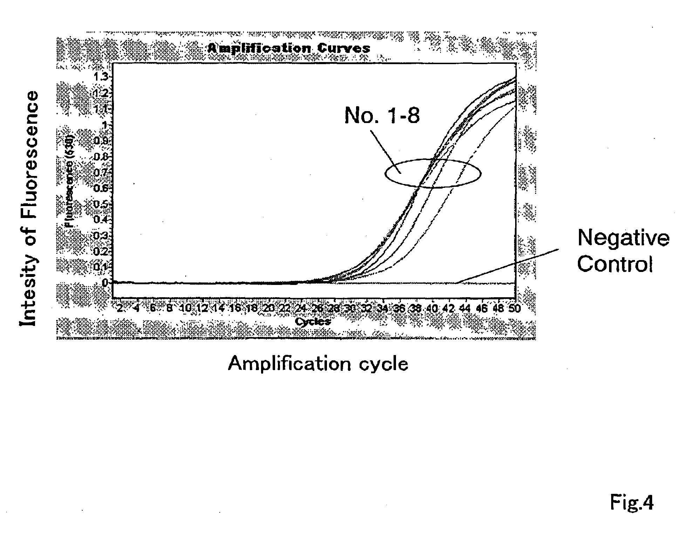

Using the DNA of the bacterial strains indicated below instead of the S. aureus DNA used in Example 1, mixed primer sets were used under the same conditions. The DNA amplified in a LightCycler (Roche) capillary tube was monitored as the rise in CYBR Green fluorescence intensity. The results are shown in FIG. 4.

### Strains Used:

| | |
|---|---|
| 1. Campylobacter jejuni | GTC 0259 type strain |
| 2. Vibrio cholerae | GTC 0037 01 E1 Tol type |
| 3. Vibrio parahaemolyticus | GTC 0056 (= CDC A3308) |
| 4. Escherichia coli | GTC 1061 0157 (VT1 and VT2 positive) |
| 5. Listeria monocytogenes | GTC 0149 (= ATCC15313) type strain |
| 6. Salmonella enterica var. Typhimurium | GIFU 11566 ST-1 |
| 7. Yersinia enterocolitica | GTC 0127 type strain |
| 8. Clostridium perfringens | GTC 0785 (= ATCC13124) |

As shown in FIG. 4, it was confirmed that the DNA of each bacteria is amplified by a species-specific primer set in the cocktail primer solution A. This demonstrated that the respective primers are able to amplify specific sequences of the DnaJ gene on the intended organisms without mutual interference.

### Example 3

Amplification of DnaJ gene of Vibrio spp.
Primer solutions obtained by mixing the forward primer common to bacteria of the genus Vibrio (SEQ ID NO: 43) and reverse primers specific to others of the following species within the same genus were prepared as indicated below.

Primer common to genus Vibrio (SEQ ID NO: 43) 2 µM

| | |
|---|---|
| Vibrio cholerae (SEQ ID NO: 44) | 1 µM |
| Vibrio mimicus (SEQ ID NO: 47) | 1 µM |
| Vibrio vulnificus (SEQ ID NO: 53) | 1 µM |
| Vibrio fluvialis (SEQ ID NO: 134) | 1 µM |
| Vibrio parahaemolyticus (SEQ ID NO: 50) | 1 µM |
| Vibrio alginolyticus (SEQ ID NO: 58) | 1 µM |

In addition, these primer solutions were used as follows to prepare PCR reaction solutions.

| | |
|---|---|
| Cocktail primer solution (containing 2 µM of forward primer and 1 µM of each of the reverse primers): | 2 µL |
| 2 x CYBER premix Hotstart Ex Taq (Takara): | 10 µL |
| DNA of each Vibrio species (solitary) | 5 ng |

The total volume was brought up to 20 µL with water.

Strains of Vibrio Bacteria Used:
Vibrio cholerae: GTC 0037 01 E1 Tol type
Vibrio mimicus: GTC 0334 type strain
Vibrio vulnificus: GTC 0116 type strain
Vibrio fluvialis: GTC 0315 type strain
Vibrio parahaemolyticus: GTC 0056 (= CDC A3308)
Vibrio alginolyticus: GTC 0057 (= ATCC17449) type strain

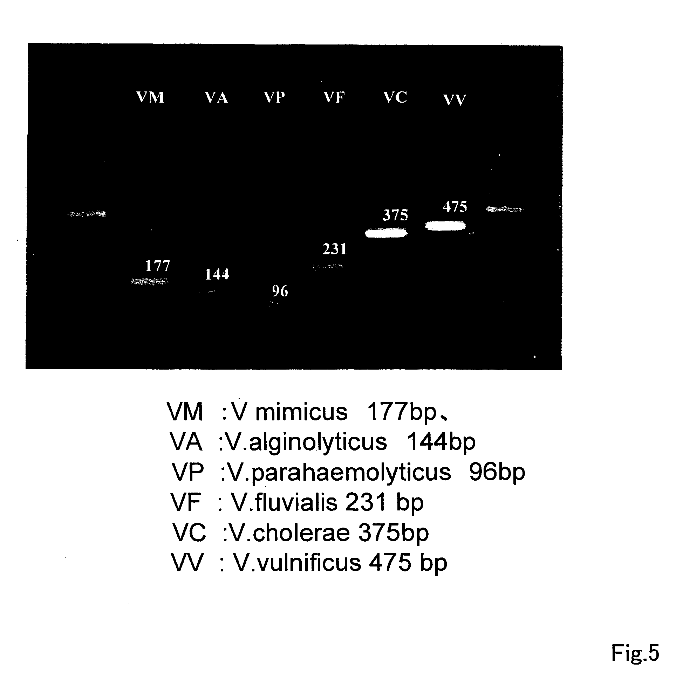

Each of the above PCR solutions was placed in a LightCycler (Roche) capillary tube along with reagents, and gene amplification (50 cycles, each consisting of 1 second at 95°C, 1 second at 55°C and 10 seconds at 72°C) was carried out with LightCycler. The results of confirming PCR amplified products by agarose electrophoresis are shown in FIG. 5.

As shown in FIG. 5, amplified products of the predicted sizes were obtained for the respective species. This demonstrated that the respective primers were able to amplify specific sequences of the DnaJ gene on the intended organisms without mutual interference.

### Example 4

### Sensitivity of Detection by Amplification of DnaJ Gene in Vibrio Bacteria, and Specific Amplification

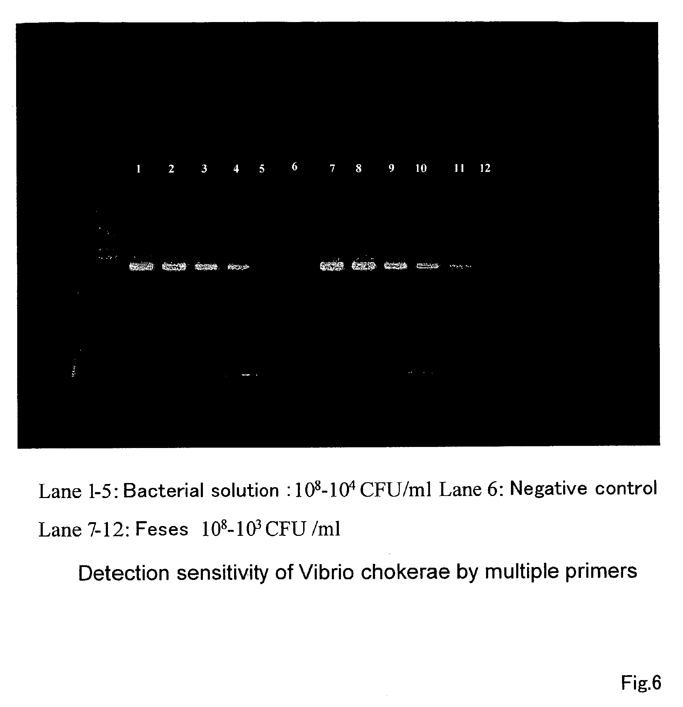

The detection sensitivity for the Vibrio cholerae TGC 0037 strain using the primer solution prepared in Example 3 was compared using samples diluted with feces. A bacterial solution of Vibrio cholerae GTC 0037 strain was mixed with physiological saline or with a 10% feces suspension, and the DNA was extracted by the phenol-chloroform method. The extracted nucleic acid was serially diluted, a PCR reaction was carried out using this nucleic acid instead of the DNA in the PCR reaction solution in Example 3, and the gene amplification sensitivity was compared by electrophoresis. The results are shown in Fig. 6.

As shown in FIG. 6, when the DNA was quantitatively diluted and the detection sensitivity was measured, the amplified product was detected at the detection levels shown in the diagram. Also, when the DNA of Vibrio cholerae was mixed with DNA extracted from normal human feces and the detection sensitivity determined, the detection sensitivity was confirmed to be at the same level as when dilution was carried out instead with distilled water. From the above, it was found that each of the primer sets in this primer solution is able to specifically amplify a specific region of the DnaJ gene of the respective intended bacterial species, even in the presence of foreign matter from feces, and that the bacterial species can be detected to a high sensitivity.

### Example 5

### DnaJ Gene Amplification and Identification of Sexually Transmitted Disease-Causing Species

Primer solutions were prepared by mixing primers designed based on specific regions of the DnaJ gene in bacterial species used in diagnosing sexually transmitted diseases.
Primers for Mycoplasma genitarium (SEQ ID NOS: 71, 72)
Primers for Chlamydia trachomatis (SEQ ID NOS: 75, 76)
Primers for Neisseria gonorrhoae (SEQ ID NOS: 79, 80)
With regard to the following species, primers were designed based on Specific regions of genes other than the DnaJ gene.
Primers for Ureaplasma spp. (Tuf gene)
SEQ ID NO: 147: GGATGGTGCAATCTTAGTTATT
SEQ ID NO: 148: ACTTGACGCGCTAATAGG
Primers for Treponema pallidum (16S rDNA gene)
SEQ ID NO: 149: TGGGGATAGCCTCTAGAAATAG
SEQ ID NO: 150: CCCTTTCCTCTCAAAGACCT
Primers for Herpes simples virus (Glycoprotein B protein gene)
SEQ ID NO: 151: CTGGTCAGCTTTCGGTACG
SEQ ID NO: 152: CCGCAGCTCGTTGTTCTC
Primers for Papilloma virus (protein E6 gene)
SEQ ID NO: 153: TCAAAAGCCACTGTGTCCT
SEQ ID NO: 154: CGACCCCTTATATTATGGAATCTT
Primers for Candida albicans (26S rDNA gene)
SEQ ID NO: 155: TCAGTAGCGGCGAGTGAA
SEQ ID NO: 156: GCCCAAAGATACCTTCTTCAAAT
Primers for Trichomonas vaginalis (18S rDNA gene)
SEQ ID NO: 157: GAAATCATAGTTCTTGGGCTCTG
SEQ ID NO: 158: CCTTCCGTCAATTCCTTCAAG

A PCR reaction solution was prepared by mixing each of the above primers in the following way.

| | |
|---|---|
| Primer solution (containing 1 µM of each primer): | 2 µL |
| 2 x CYBER premix Hotstart Ex Taq (Takara): | 10 µL |
| DNA of each strain | 5 ng |

The total volume of the above mixture was brought up to 20 µL with water.

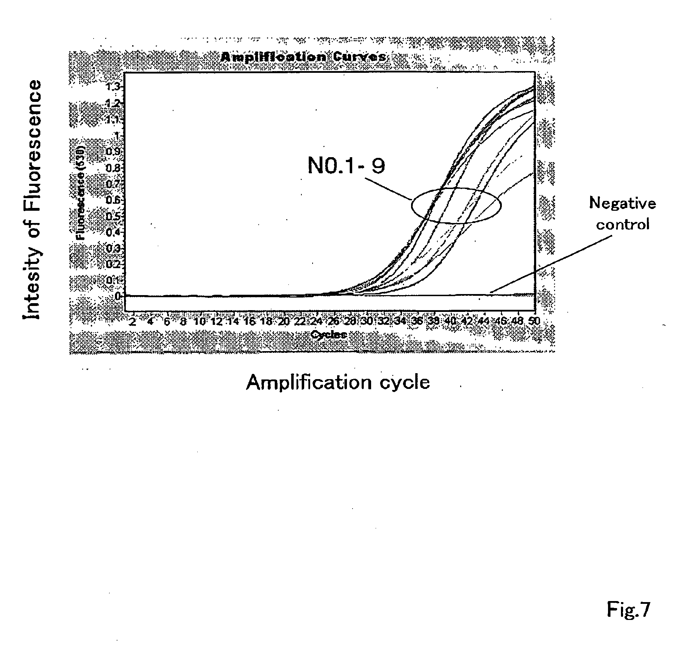

The above PCR solutions were placed in a LightCycler (Roche) capillary tube along with reagents, and gene amplification (50 cycles, each consisting of 1 second at 95°C, 1 second at 55°C and 10 seconds at 72°C) was carried out using LightCycler. The strains indicated below were used in this experiment. FIG. 7 shows the results obtained by monitoring the DNA amplified in the LightCycler (Roche) capillary tube as the rise in CYBR Green fluorescence intensity.

Strains Used:

| | |
|---|---|
| 1. Mycoplasma genitarium: | GGS 2289 (Clone from clinical sample) |
| 2. Chlamydia trachomatis: | GGS 922 (Clone from clinical sample) |
| 3. Neisseria gonorrhoae: | GTC 02085 (= ATCC 49226) |
| 4. Ureaplasma spp.: | GGS 1699 (Clone from clinical sample) |
| 5. Treponema pallidum: | GGS 745 (Clone from clinical sample) |
| 6. Herpes simplex virus | GGS 1943 (Clone from clinical sample) |
| 7. Papilloma virus | GGS 1944 (Clone from clinical sample) |
| 8. Candida albicans | GTC 00654 (= IFM 5801) |
| 9. Trichomonas vaginalis | GGS 1941 (Clone from clinical sample) |

GTC: Gifu Type Culture Collection
GGS: Gifu Recombinant Strain Collection
ATCC: Americana Type Culture Collection, USA
NCTC: National Collection of Type Culture, UK
IFM: Research Center for Pathogenic & Microbial Toxicoses, Chiba University)

As shown in FIG. 7, the DNA of each strain was amplified. This demonstrated that the respective primers are able to amplify specific sequences of the DnaJ gene on the intended organisms without mutual interference.

### Example 6

### Amplification and Identification of DnaJ Gene of Pneumonia and Pharyngitis-Causing Pathogen Species

A primer solution was prepared by mixing together primers designed based on a specific region of the DnaJ gene in species of pathogens which cause pneumonia and pharyngitis.
Primers for Streptococcus pneumoniae (SEQ ID NOS: 9,10)
Primers for Streptococcus pyogenes (SEQ ID NOS: 1, 2)
Primers for Staphylococcus aureus (SEQ ID NOS: 13, 14)
Primers for Klebsiella pneumoniae (SEQ ID NOS: 17, 18)
Primers for Escherichia coli (SEQ ID NOS: 21, 22)
Primers for Mycobacterium tuberculosis (SEQ ID NOS: 25, 26)
Primers for Chlamydia pneumoniae (SEQ ID NOS: 39, 40)
Primers for Legionella pneumophila (SEQ ID NOS: 35, 36)
Primers for Mycoplasma pneumoniae (SEQ ID NOS: 31, 32)
Primers for the Mycobacterium group (SEQ ID NOS: 29, 30)

Strains Used:

| | |
|---|---|
| Streptococcus pneumoniae | GTC 01147 (= NCTC 7465) |
| Streptococcus pyogenes | GTC 01839 |
| Staphylococcus aureus | GTC 02805 |
| Klebsiella pneumoniae | GTC 00107 (= NCTC 9633) |
| Escherichia coli | GTC 09975 (= ATCC 25 868) |
| Mycobacterium tuberculosis | GTC 12850 (= ATCC 27294) |
| Chlamydia pneumoniae | ATCC VR 145 |
| Legionella pneumophila | GTC 00702 (= ATCC 33737) |
| Mycoplasma pneumoniae | GTC 00666 (= ATCC 15531) |
| Mycobacterium avium | GTC 00603 (=ATCC 25291) |

GTC: Gifu Type Culture Collection
GGS: Gifu Recombinant Strain Collection
ATCC: Americana Type Culture Collection, USA
NCTC: National Collection of Type Culture, UK
IFM: Research Center for Pathogenic & Microbial Toxicoses, Chiba University)

A PCR reaction solution was prepared by mixing each of the above primers in the following way.

| | |
|---|---|
| Primer solution (containing 1 µM of each primer): | 2 µL |
| 2 × CYBER premix Hotstart Ex Taq (Takara): | 10 µL |
| DNA of each strain | 5 ng |

The total volume of the above was brought up to 20 µL with water.

The above PCR solutions were each placed in a LightCycler (Roche) capillary tube along with reagents, and gene amplification (50 cycles, each consisting of 1 second at 95°C, 1 second at 55°C and 10 seconds at 72°C) was carried out using LightCycler. DNA of the above strains were used in this experiment. On checking the PCR amplified products by agarose electrophoresis, it was found that these primer sets are able to specifically amplify a specific region of the DnaJ gene of the respective intended species, and are able to detect the species at a high sensitivity.

### Example 7

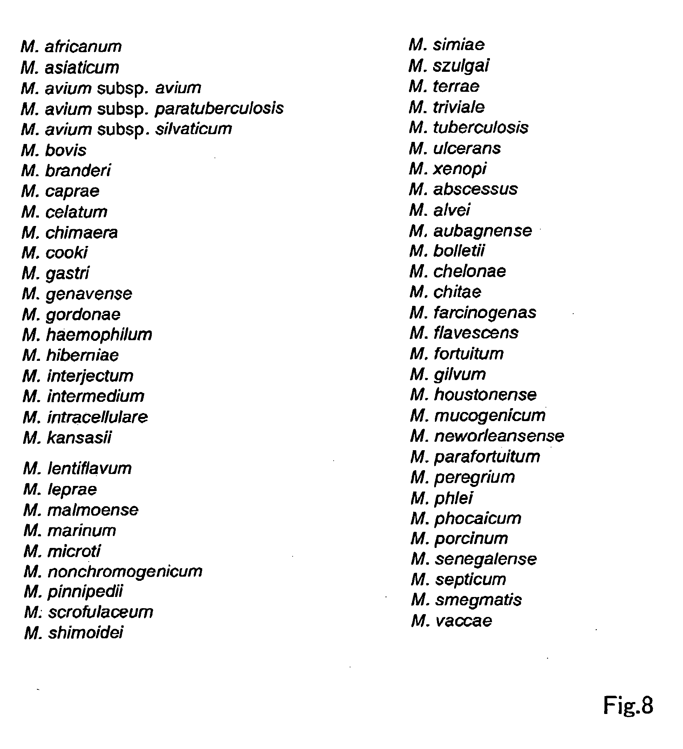

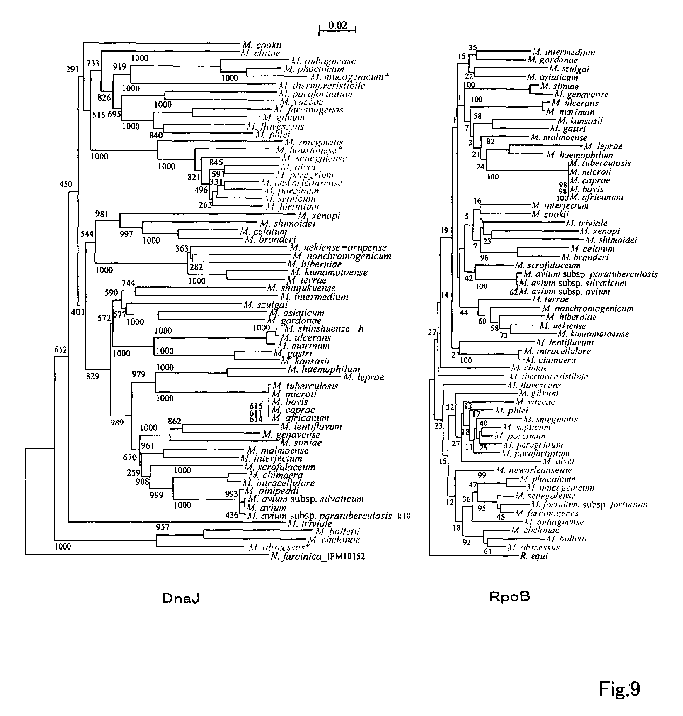

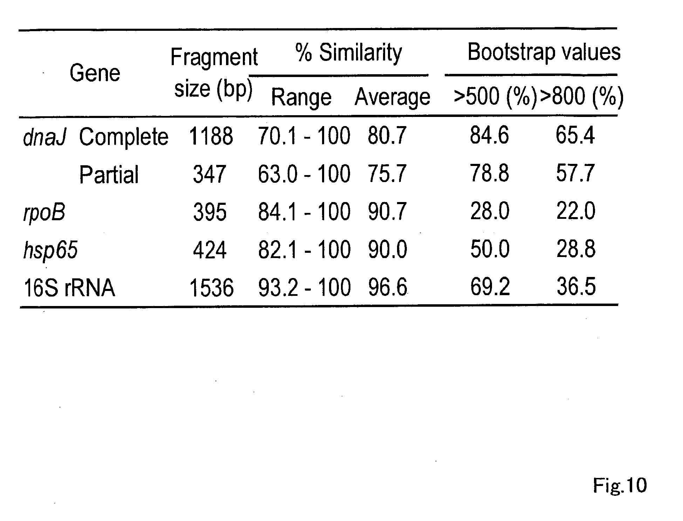

Amplification of DnaJ Gene in Mycobacterium Spp., and Identification by Sequencing Substantially the entire region of the DnaJ gene in 55 species and 3 subspecies type strains of genus Mycobacterium bacteria (shown in FIG. 8) was sequenced by the Primer working method (Applied Biosystem), and a 386 bp amplified sequence obtained with SEQ ID NOS: 29, 30, 131, 132 and 133 was compared with the 1,188 bp sequence of substantially the entire region. These results were compared with the existing gene RpoB for which analysis has already been carried out. The results are shown in FIGS. 9 and 10.

As shown in FIGS. 9 and 10, the 16S rDNA sequences had an average similarity of 96.6%, whereas the average similarity for DnaJ gene sequences was 80.7%. The corresponding values for the rpoB gene and the hsp65 gene were 90.0% and 90.7% respectively, indicating that the DnaJ gene had the highest degree of polymorphism.

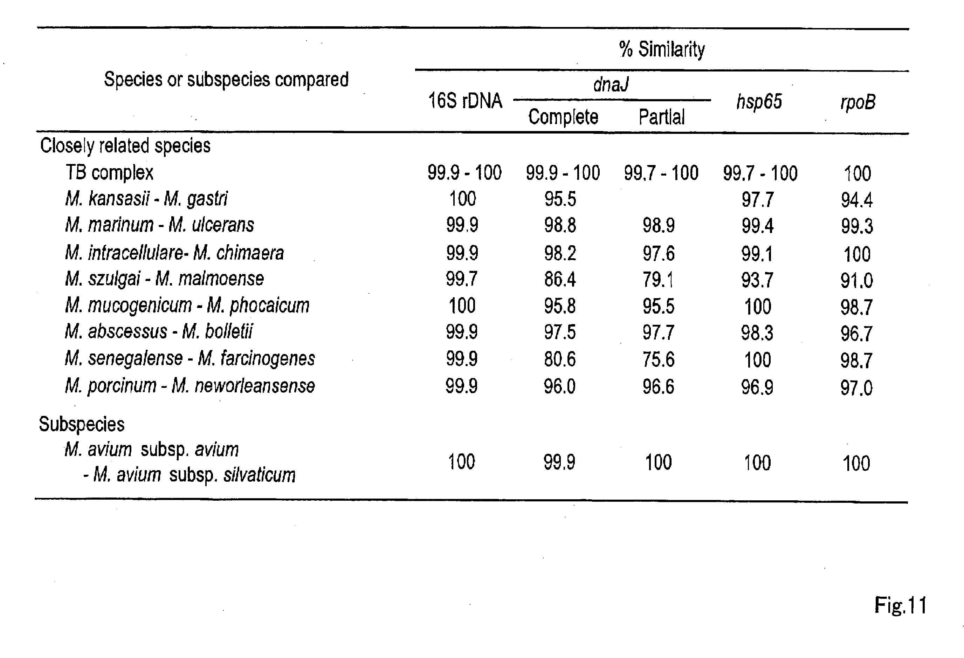

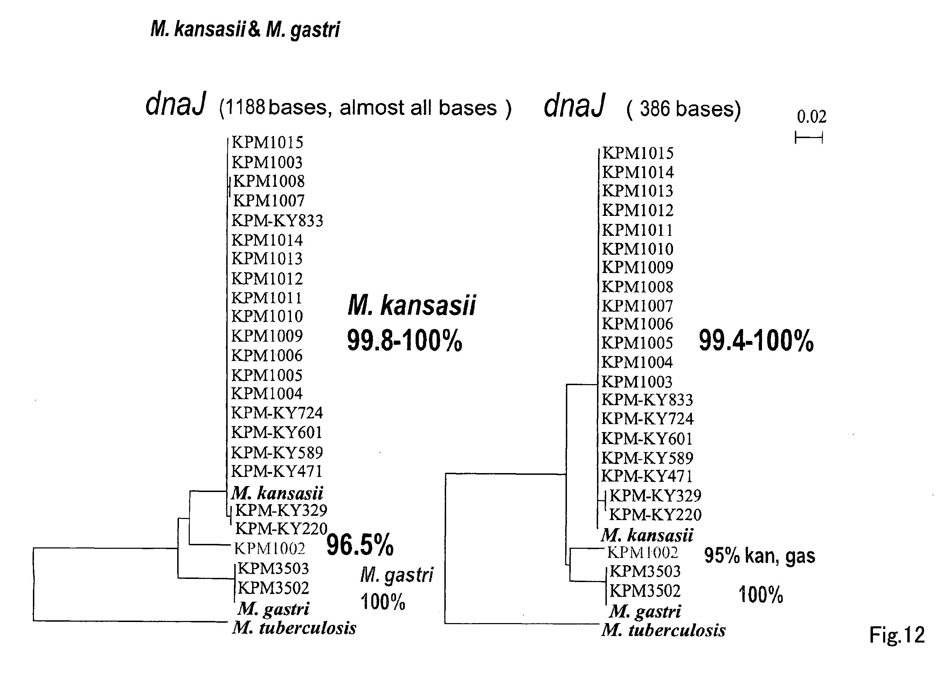

Also, in M. kansasii and M. gastri, the respective 16S rDNA sequences were 100% in agreement, making them indistinguishable (FIG. 11), whereas a comparison of the DnaJ genes of each showed a similarity of 95.5%, indicating some difference therebetween. Accordingly, based on the biochemical properties, the above-mentioned SEQ ID NOS. 29, 30, 131, 132 and 133 of 23 clinically isolated strains (KPM number strains) of M. kansasii (20 strains) and M. gastri (2 strains) were amplified, and a phylogenetic tree was prepared using the sequences of the complete type strains alrcady sequenced. As shown in FIG. 12, the results for all the strains agreed with the results obtained by identification based on biochemical properties.

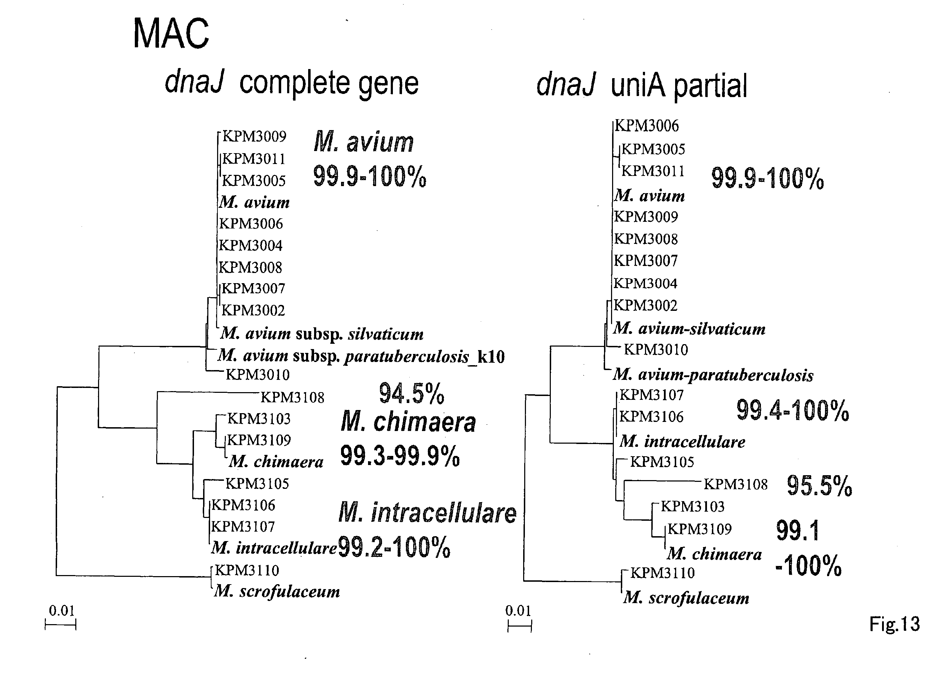

Also, 16 clinically isolated strains (the KPM strains in the diagram), referred to collectively as the M. avium - M. intracellulare group (MAC group) because of the difficulty in distinguishing M. avium and M. intracellulare for having similar biochemical properties, were similarly sequenced and compared. Those results are shown in FIG. 13.

As shown in Fig. 13, nine strains were M. avium, three strains were M. intracellular, three strains were newly classified M. chimaera, and one strain was identified as M. scroflaceum. From the above, this method of identification using the complete base sequence and partial sequences was found to be effective for accurately identifying a group of bacteria that are indistinguishable based on their biochemical properties.

### Example 8

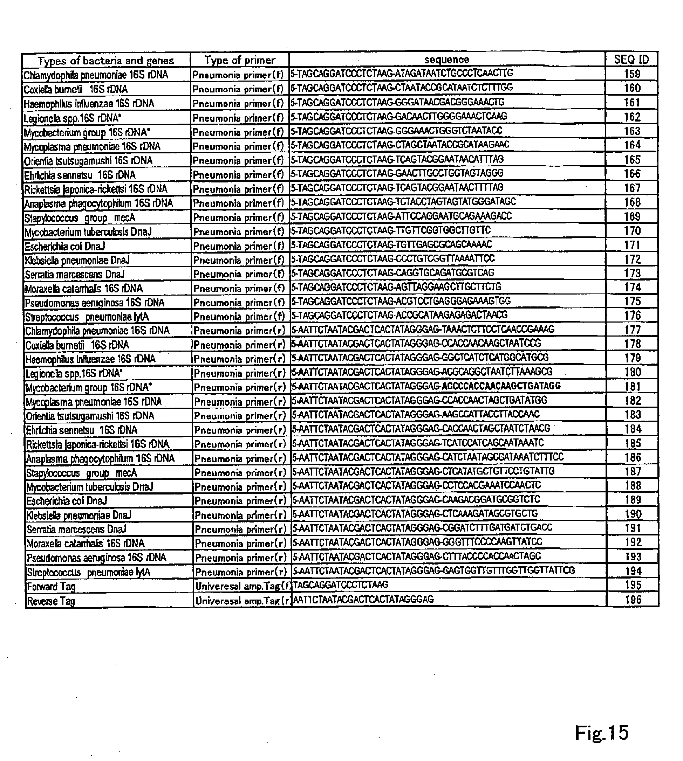

In the present example, multiplex PCR was carried out using Legionella pneumophila GTC 1279 DNA as the target nucleic acid. The primers were furnished for use in the form of a mixture of 18 sets of tagged annealing primers (forward primers (SEQ ID NOS: 1 to 18) and reverse primers (SEQ ID NOS: 19 to 36) (both with tags) at respective final concentrations of 0.1 µM), and a mixture of a set of amplification primers having tags with the same sequences as the respective tags on the annealing primers (the latter mixture containing a forward primer (SEQ ID NO: 37) and a reverse primer (SEQ ID NO: 38) (both composed only of a tag) at respective final concentrations of 1 µM). The sequences of these primers are shown in FIG. 15.

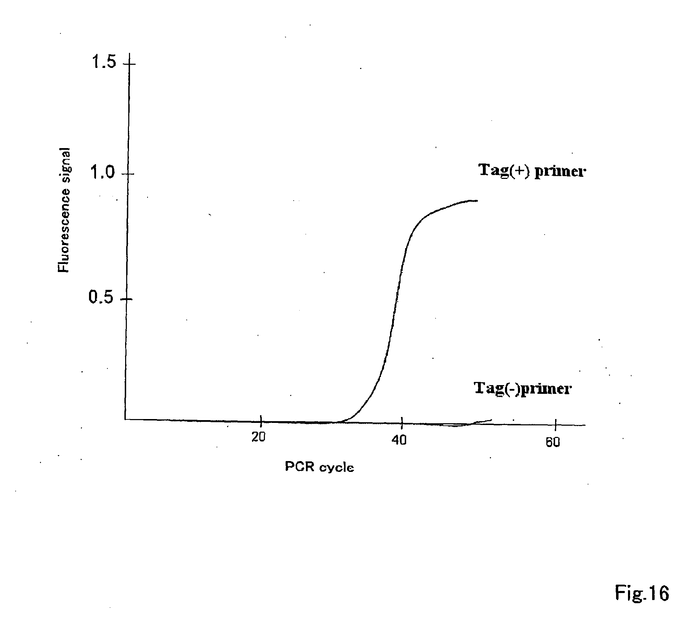

Using these primer mixtures and a DNA solution (Legionella pneumophila GTC 1279 DNA, 1 µg/mL), a PCR reaction solution of the composition shown below was prepared, and a PCR reaction was carried out using LightCycler (Roche) under the following conditions. As a comparative example, a mixture of untagged annealing primers containing annealing primers in which the 18-base tag sequences had been removed from the 5' ends of the forward primers in the above-mentioned 18 primer sets and containing annealing primers in which the 27-base tag sequences had been removed from the 5' ends of the reverse primers (which mixture had final concentrations of each of the forward primers and reverse primers (all untagged) of 0.1 µM) was furnished and used to prepare a PCR reaction solution of the following composition, and a PCR reaction was carried out in the same way as in the above working example of the invention. FIG. 16 shows the results obtained on monitoring amplification.

Composition of PCR Reaction Solution in Example 8:

| | |
|---|---|
| 2 x SYBR premix Ex Taq (TaKaRa) | 10 µL |
| Primer mixture (1 µM each of F primer, R primer): | 2 µL |
| Amplification primer mixture (10 µM each of F primer, R primer) | 2 µL |
| Legionella pneumophila GTC 1279 DNA (1 ng/mL) | 1 µL |
| dH₂O | 5 µL |
| Total volume | 20 µL |

Composition of PCR Reaction Solution in Comparative Example:

| | |
|---|---|
| 2 × SYBR premix Ex Taq (TaKaRa) | 10 µL |
| Primer mixture (1 µM each of F primer, R primer): | 2 µL |
| Legionella pneumophila GTC 1279 DNA (1 ng/mL) | 1 µL |
| dH₂O | 7 µL |
| Total volume | 20 µL |

### Reaction Conditions:

Sixty cycles, each cycle consisting of 1 second at 95°C, 1 second at 60°C and 5 seconds at 72°C.

As shown in FIG. 16, with the tagged primer sets and amplification primer sets of Example 8, Legionella pneumophila GTC 1279 DNA started to show an increase in amplification signals after about 35 cycles, and exhibited good detection sensitivity after about 40 cycles. In the comparative example in which amplification primer was not used, even after 50 cycles, no amplification of the target nucleic acid whatsoever was observed. The above results demonstrate that, by using an annealing primer which has both an annealing sequence that selectively anneals to a target nucleic acid and a tag sequence and by using also an amplification primer which has the same tag sequence as the tag sequence of the annealing primer, specific target nucleic acids can be fully amplified even when a plurality of types of primer sets are used.

### Example 9

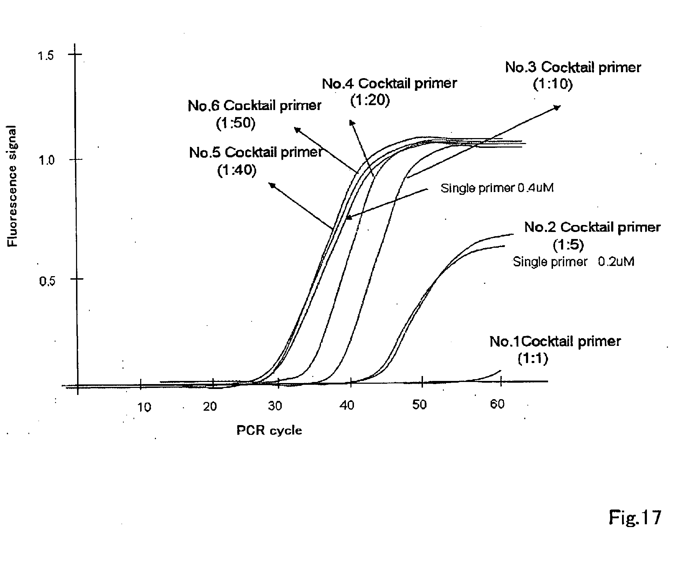

In this example, the mixing ratio of tagged annealing primers to amplification primers prepared in Example 8 was varied from 1:1 to 1:50, as shown in Table 2. A PCR reaction solution was prepared as shown below, and PCR was carried out under the following conditions. Mycoplasma pneumoniae GTC 2987 DNA was used as the target nucleic acid. As a comparative example, PCR reaction solutions of the compositions indicated below were prepared using prior-art single primer solutions (4 µM, 8 µM), and PCR was carried out under the same conditions as in the working example. The results are shown in FIG. 17. The single primers were the Mycoplasma pneumoniae 16S rDNA forward primer and reverse primer from which the tag sequences had been removed.

**Table 2**

| | Primer mixture | | Mixing ratio | |
|---|---|---|---|---|
| No. 1 | Annealing 0.5 µM: 2 µL | Amplification Tag (0.5 µM) | 2 µL | 1:1 |
| No. 2 | Annealing 0.5 µM: 2 µL | Amplification Tag (2.5 µM) | 2 µL | 1:5 |
| No. 3 | Annealing 0.5 µM: 2 µL | Amplification Tag (5 µM) | 2 µL | 1:10 |
| No. 4 | Annealing 0.5 µM: 2 µL | Amplification Tag (10 µM) | 2 µL | 1:20 |
| No. 5 | Annealing 0.5 µM: 2 µL | Amplification Tag (20 µM) | 2 µL | 1:40 |
| No. 6 | Annealing 0.5 µM: 2 µL | Amplification Tag (25 µM) | 2 µL | 1:50 |

Composition of PCR Reaction Solution in Example 9:

| | |
|---|---|
| 2 × SYBR premix Hot Start (TaKaRa) | 10 µL |
| Mycoplasma pneumoniae GTC 2987 DNA (1 ng/mL) | 1 µL |
| Primer mixture | 4 µL |
| dH₂O | 5 µL |
| Total volume | 20 µL |

Composition of PCR Reaction Solution in Comparative Example:

| | |
|---|---|
| 2 × SYBR premix Hot Start (TaKaRa) | 10 µL |
| Mycoplasma pneumoniae GTC 2987 DNA (1 ng/mL) | 1 µL |
| Single primer* (4 µM, 8 µM) mixture | 4 µL |
| dH₂O | 5 µL |
| Total volume | 20 µL |

### Reaction Conditions:

Sixty cycles, each cycle consisting of 1 second at 95°C, 1 second at 60°C and 5 seconds at 72°C.

As shown in FIG. 17, as the ratio of amplification primers to annealing primers rose, the amplification efficiency increased. When this ratio became 10 or more, an amplification efficiency was achieved which was comparable to that when a solitary primer was used at a concentration of 0.4 µM and amplified.

### Example 10

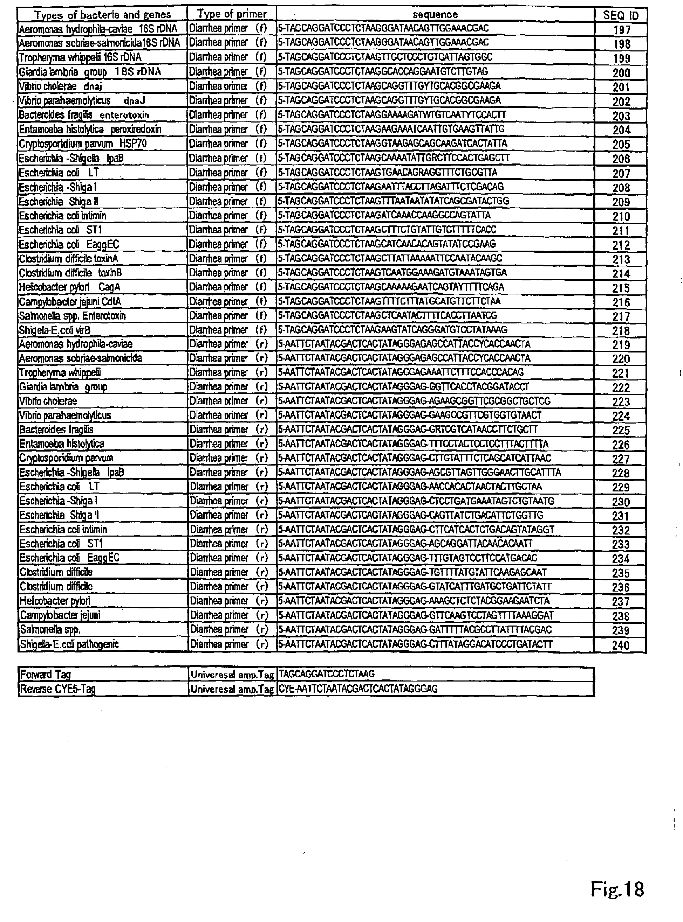

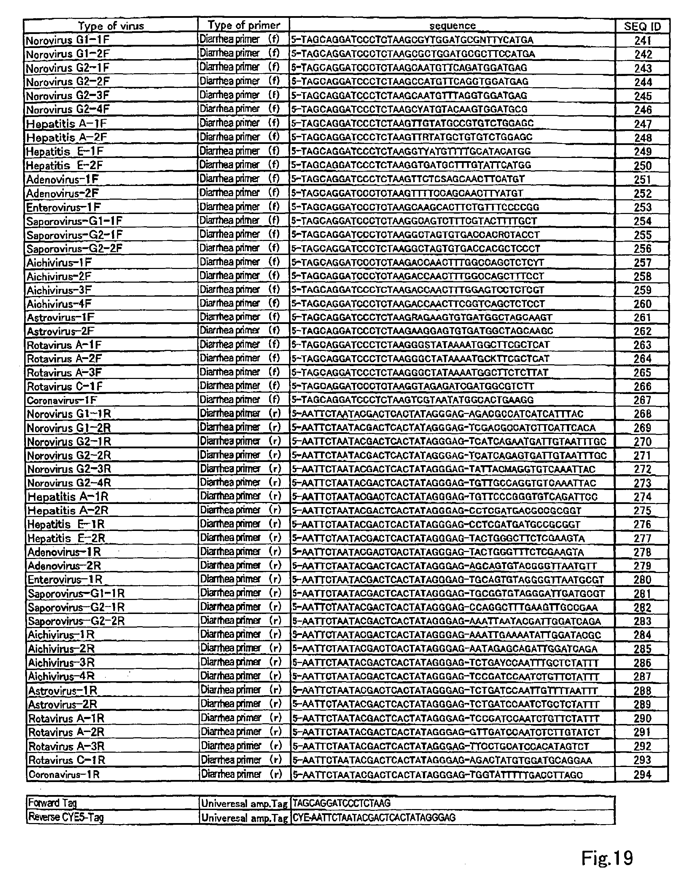

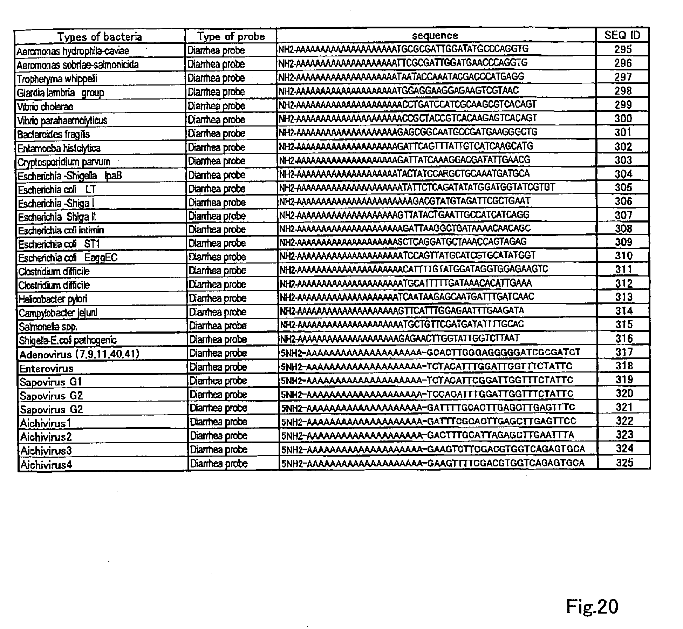

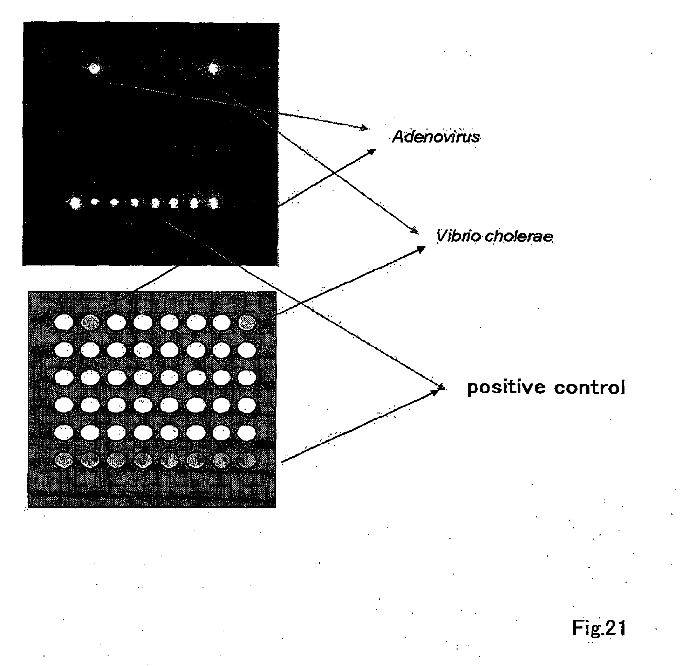

In this example, PCR reaction solutions were prepared in the following compositions by setting the mixing ratio of the annealing primers and amplification primers whose target nucleic acids are specific regions of genes possessed by the pathogenic bacteria and viral pathogens causing diarrhea shown in FIGS. 18 and 19 to 1:50, and PCR was carried out under the reaction conditions shown below. Following PCR, the PCR products were hybridized at 55°C for 30 minutes on a silicon chip (Toyo Kohan Co., Ltd.) on which the probes shown in FIG. 20 had been placed. Following hybridization, the chip was rinsed, and the Cye5 fluorescence intensity was measured with a laser scanner (Scan Array 4000, available from GSI Lumonics). The results are shown in FIG. 21. FIG. 21 also shows positional information on the spotted probes.

Composition of PCR Reaction Solution in Example 10:

| | |
|---|---|
| 2 × SYBR premix Hot Start (TaKaRa) | 10 µL |
| Vibrio cholerae DNA (1 ng/mL) | 1 µL |
| Adenovirus DNA (1 ng/mL) | 1 µL |
| Annealing primer mixture (0.2 µM each) | 2 µL |
| Amplified primer mixture (10 µM each) | 2 µL |
| dH₂O | 4 µL |
| Total volume | 20 µL |

### Reaction Conditions:

Sixty cycles, each cycle consisting of 1 second at 95°C, 1 second at 60°C and 5 seconds at 72°C.

As shown in FIG. 21, even when 49 types of annealing primer sets were included, it was possible to detect each of two types of target nucleic acids specifically and to a good sensitivity.

### Example 11

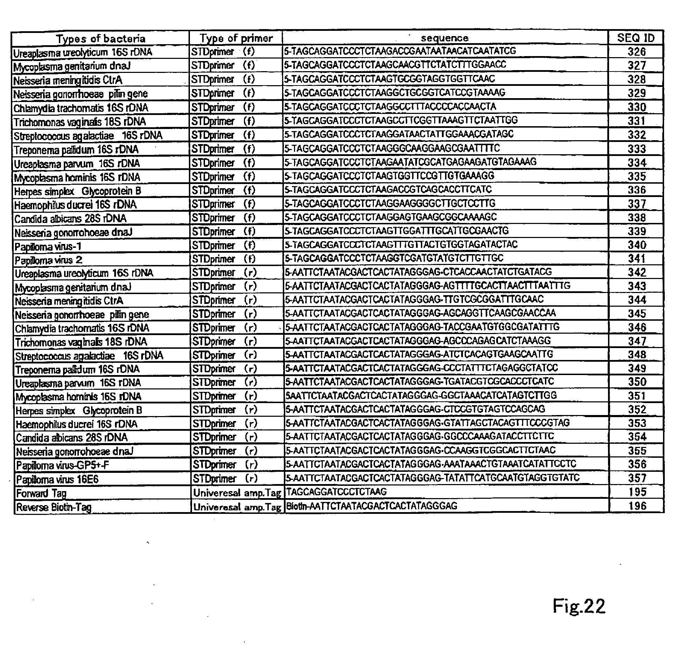

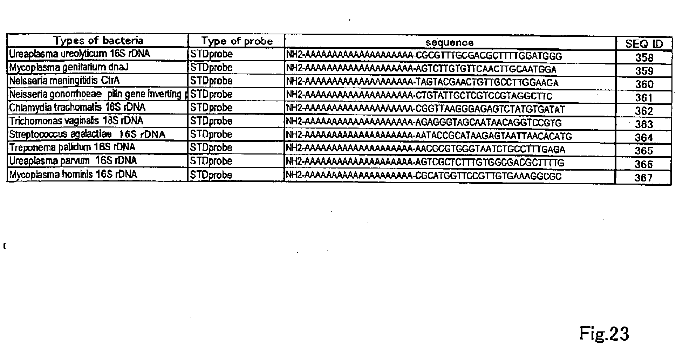

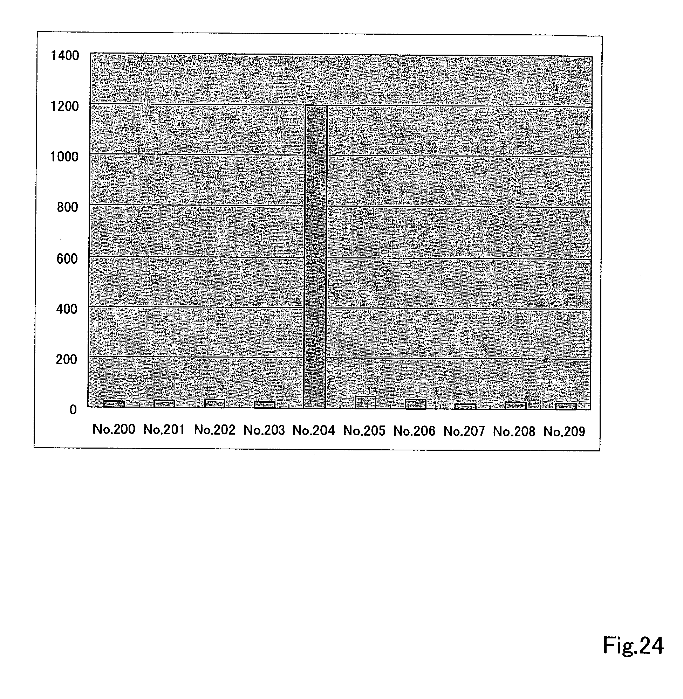

In this invention, a mixture of the 16 types of annealing primer sets for sexually transmitted diseases (final concentration of each primer, 0.02 µM) shown in FIG. 22 and a mixture of amplification primer sets (each 10 µM) were furnished, a PCR reaction solution of the composition shown below was prepared in such a way as to set the ratio of amplification primer to annealing primer to 20, and PCR was carried out under the following conditions. Following the amplification reaction, fluorescent beads (available from Luminex Corporation) to which the probes shown in FIG. 23 had been bonded and streptavidin-phycoerythrin was added to and reacted with the PCR product, and the fluorescence intensity was measured using a Luminex 100 fluorescence measuring system (Luminex Corporation). The results are shown in FIG. 24.

As shown in FIG. 24, the single target nucleic acid included in the PCR reaction solution bonded at a high specificity with the probe for Chlamydia trachomatis. From the above, this target nucleic acid was found to have been specifically amplified within the PCR product.

### Example 12

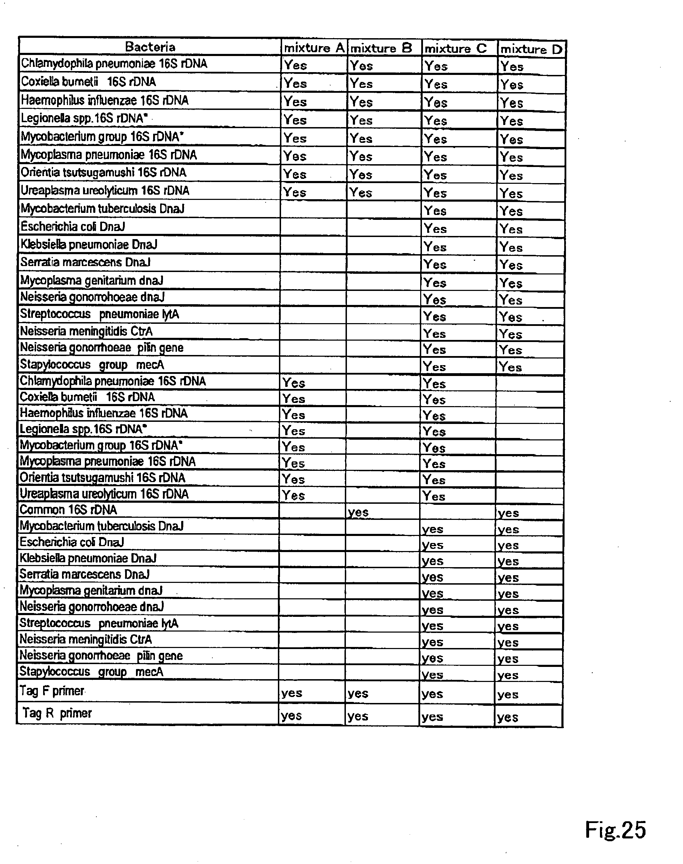

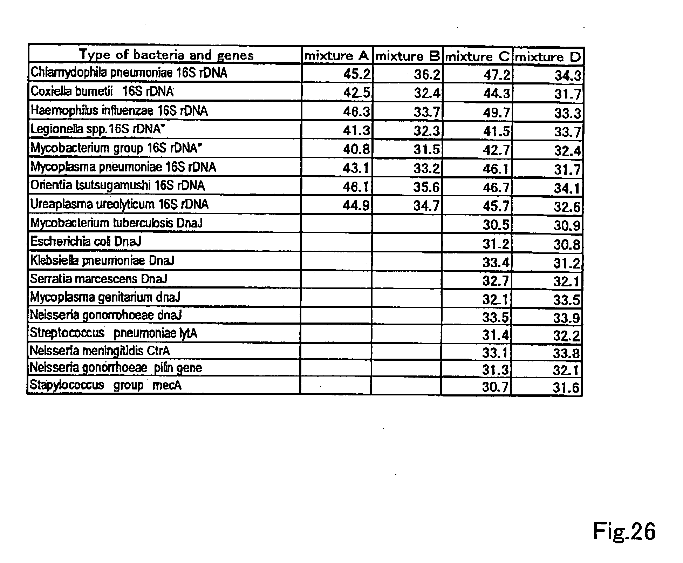

In this example, the four types of cocktail primers shown in FIG. 25 were prepared. Each of the primers shown in FIG. 25 and used in this example was primers having a sequence that has already been mentioned. The primer PCR reaction solution was prepared so as to include 200 pg/mL of DNA from each of the species shown in FIG. 25, 0.075 µM of each of the annealing primers, 0.8 µM of each of the amplification primers, and 0.2 µM of the common 16S RNA primer. The DNA polymerase used was SYBR premix Hot Start (TaKaRa). Using this PCR reaction solution, 50 cycles of the PCR reaction were carried out, each cycle consisting of 1 second at 95°C, 1 second at 55°C and 10 seconds at 72°C, after one minute heating at 95°C. The results (CTm values) are shown in FIG. 26. The CTm value is the number of PCR cycles up until a signal, such as the fluorescent signal intensity, reaches the half-peak value of the amplification curve when differentiated once. A smaller CTm value indicates a better amplification efficiency.

As shown in FIG. 26, when PCR was carried out with the 16S rRNA gene as the target nucleic acid and using cocktail primer A composed of annealing primers and amplification primers, the CTm value was about 40 to 46. By contrast, with cocktail primer B in which a reverse primer served as the common sequence of 16S rRNA, a decreasing trend in the CTm value was observed. That is, by designing an annealing primer using 16S rRNA as the target nucleic acid, even when the primers of eight types of phylogenically differing species of bacteria were mixed, the amplification efficiency tended to decrease even with respect to the control example. Therefore, 16S rRNA was found to be unsuitable as the target nucleic acid for multiplex PCR such as is used in the present invention.

By contrast, as is apparent from the results obtained for cocktail primers C and D, when annealing primers and amplification primers were used with the DnaJ gene serving as the target nucleic acid, even with the presence of many other primers (total of 18 types), the primers annealed specifically to the target sequence, enabling efficient amplification. Therefore, the DnaJ gene was found to be beneficial as the target nucleic acid for such multiplex PCR.

### Sequence Table Text

SEQ ID NOS: 1 to 86, 131, 132, 133, 135 to 158, 159 to 294 and 326 to 357: primers SEQ ID NOS: 87 to 130,134,295 to 325 and 358 to 367: probes

## Claims

1. A process for detecting species of bacteria, in which two or more species of bacteria selected from among bacteria of the genera Staphylococcus, Streptococcus, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia are subject of detection, the process comprising:
(a) a step of contacting a test sample which may contain nucleic acids of subject bacteria with two or more types of nucleic acid molecules which hybridize with at least portions ofDnaJ genes in the two or more subject bacterial species; and
(b) a step of detecting whether the nucleic acid molecules have hybridized with nucleic acids within the test sample.

2. The detection process of claim 1, wherein the two or more species of bacteria are selected from among Streptococcus pyogenes, Streptococcus agalactiae, Streptococcus pneumoniae, Staphylococcus aureus, Klebsiella pneumoniae, Escherichia coli, Mycobacterium tuberculosis, Mycoplasma pneumoniae, Mycoplasma genitarium, Vibrio cholerae, Vibrio parahaemolyticus, Vibrio vulnificus, Vibrio fluvialis, Vibrio alginolyticus, Neisseria meningitides, Neisseria gonorrhoae, Bacillus cereus, Chlamydia trachomatis, Chlamydophila pneumoniae and Campylobacter jejuni.

3. The process of claim 1 or 2, wherein the subject of detection is the two or more species of bacteria selected from among bacteria of the genera Klebsiella, Escherichia, Vibrio, Bacillus, Neisseria, Campylobacter, Chlamydia, Chlamydophila, Mycoplasma, Listeria, Salmonella and Yersinia.

4. The detection process of any of claims 1 to 3, wherein the nucleic acid molecules include one or more species-specific base sequences extracted by aligning base sequences of the DnaJ gene of strains belonging to the same species, or one or more base sequences substantially identical therewith.

5. The detection process of any of claims 1 to 4, wherein the step (a) performs a polymerase chain reaction using at least one type of a first primer set having a tag sequence and a base sequence which selectively anneals to a portion of the DnaJ gene and at least one type of a second primer set having a tag sequence which is substantially the same as the tag sequence of the first primer set, and performs an amplification of at least a portion of the DnaJ gene with the first primer set and an amplification of an amplified product obtained by the amplification with the first primer set with the second primer set.

6. The amplification process of claim 5, wherein the polymerase chain reaction step performs a single PCR reaction cycle.

7. The detection process of any of claims 1 to 6, wherein
in a case where the subject bacteria include Streptococcus pyogenes, the nucleic acid molecules include one or more base sequences selected from among SEQ ID NOS: 1 to 4 and SEQ ID NOS: 89 and 90, or one or more base sequences substantially identical thereto;
in a case where the subject bacteria include Streptococcus agalactiae, the nucleic acid molecules include one or more base sequences selected from among SEQ ID NOS: 5 to 8 and SEQ ID NOS: 91 and 92, or one or more base sequences substantially identical thereto;
in a case where the subject bacteria include Streptococcus pneumoniae, the nucleic acid molecules include one or more base sequences selected from among SEQ ID NOS: 9 to 12 and SEQ ID NOS: 93 and 94, or one or more base sequences substantially identical thereto;
in a case where the subject bacteria include Staphylococcus aureus, the nucleic acid molecules include one or more base sequences selected from among SEQ ID NOS: 13 to 16 and SEQ ID NOS: 97 and 98, or one or more base sequences substantially identical thereto;
in a case where the subject bacteria include Klebsiella pneumoniae, the nucleic acid molecules include one or more base sequences selected from among SEQ ID NOS: 17 to 19 and SEQ ID NOS: 109 to 110, or one or more base sequences substantially identical thereto;
in a case where the subject bacteria include Escherichia coli, the nucleic acid molecules include one or more base sequences selected from among SEQ ID NOS: 21 to 24 and SEQ ID NOS: 101 and 102, or one or more base sequences substantially identical thereto;
in a case where the subject bacteria include Mycobacterium tuberculosis, the nucleic acid molecules include one or more base sequences selected from among SEQ ID NOS: 25 to 28 and SEQ ID NOS: 103 and 104, or one or more base sequences substantially identical thereto;
in a case where the subject bacteria include Mycobacterium spp. (exclusive of the M. tuberculosi group), the nucleic acid molecules include one or more base sequences selected from among SEQ ID NOS: 29 and 30 and SEQ ID NOS: 131 to 133, or one or more base sequences substantially identical thereto;
in a case where the subject bacteria include Legionella pneumophila, the nucleic acid molecules include one or more base sequences selected from among SEQ ID NOS: 35 to 38 and SEQ ID NOS: 115 and 116, or one or more base sequences substantially identical thereto;
in a case where the subject bacteria include Vibrio cholerae, the nucleic acid molecules include one or more base sequences selected from among SEQ ID NOS: 43 to 46 and SEQ ID NOS: 127 and 128, or one or more base sequences substantially identical thereto;
in a case where the subject bacteria include Vibrio mimicus, the nucleic acid molecules include one or more base sequences selected from among SEQ ID NO: 43, SEQ ID NOS: 47 to 49 and SEQ ID NOS: 125 and 126, or one or more base sequences substantially identical thereto;
in a case where the subject bacteria include Vibrio parahaemolyticus, the nucleic acid molecules include one or more base sequences selected from among SEQ ID NO: 43, SEQ ID NOS: 50 to 52, SEQ ID NOS: 123 and 124, or one or more base sequences substantially identical thereto;
in a case where the subject bacteria include Vibrio vulnificus, the nucleic acid molecules include one or more base sequences selected from among SEQ ID NO: 43, SEQ ID NOS: 53 to 55 and SEQ ID NOS: 119 and 120, or one or more base sequences substantially identical thereto;
in a case where the subject bacteria include Vibrio fluvialis, the nucleic acid molecules include one or more base sequences selected from among SEQ ID NO: 43, SEQ ID NO: 134, SEQ ID NOS: 56 and 57, and SEQ ID NOS: 117 and 118, or one or more base sequences substantially identical thereto;
in a case where the subject bacteria include Vibrio alginolyticus, the nucleic acid molecules include one or more base sequences selected from among SEQ ID NO: 43, SEQ ID NOS: 58 to 60, and SEQ ID NOS: 121 and 122, or one or more base sequences substantially identical thereto;
in a case where the subject bacteria include Bacillus cereus, the nucleic acid molecules include one or more base sequences selected from among SEQ ID NOS: 61 to 66 and SEQ ID NOS: 87 and 88, or one or more base sequences substantially identical thereto;
in a case where the subject bacteria include Campylobacter jejuni, the nucleic acid molecules include one or more base sequences selected from among SEQ ID NOS: 67 to 70 and SEQ ID NOS: 129 and 130, or one or more base sequences substantially identical thereto;
in a case where the subject bacteria include Neisseria meningitidis, the nucleic acid molecules include one or more base sequences selected from among SEQ ID NOS: 83 to 86 and SEQ ID NOS: 111 and 112, or one or more base sequences substantially identical thereto;
in a case where the subject bacteria include Neisseria gonorrohae, the nucleic acid molecules include one or more base sequences selected from among SEQ ID NOS: 79 to 82 and SEQ ID NOS: 113 and 114, or one or more base sequences substantially identical thereto;
in a case where the subject bacteria include Chlamydia trachomatis, the nucleic acid molecules include one or more base sequences selected from among SEQ ID NOS: 75 to 78 and SEQ ID NOS: 109 and 110, or one or more base sequences substantially identical thereto;
in a case where the subject bacteria include Chlamydia pneumoniae, the nucleic acid molecules include one or more base sequences selected from among SEQ ID NOS: 39 to 42 and SEQ ID NOS: 107 and 108, or one or more base sequences substantially identical thereto;
in a case where the subject bacteria include Mycoplasma genitarium, the nucleic acid molecules include one or more base sequences selected from among SEQ ID NOS: 71 to 74 and SEQ ID NOS: 105 and 106, or one or more base sequences substantially identical thereto; and
in a case where the subject bacteria include Mycoplasma pneumoniae, the nucleic acid molecules include one or more base sequences selected from among SEQ ID NOS: 31 to 34 and SEQ ID NOS: 95 and 96, or one or more base sequences substantially identical thereto.

8. A nucleic acid molecule kit for detecting and identifying species of bacteria, in which two or more species of bacteria selected from among bacteria of the genera Streptococcus, Staphylococcus, Enterococcus, Peptostreptococcus, Klebsiella, Escherichia, Enterobacter, Legionella, Neisseria, Bacillus, Helicobacter, Corynebacterium, Pseudomonas, Burkholderia, Haemophilus, Bacteroides, Enterococcus, Nocardia, Prevotella, Neisseria, Moraxella, Flavobacterium, Clostridium, Treponema, Sphingobacterium, Leptospira, Campylobacter, Listeria, Salmonella and Yersinia are subjects of detection and identification,
wherein the kit includes nucleic acid molecules which hybridize with at least portions of DnaJ genes in subject bacteria.

9. The kit of claim 8, wherein the nucleic acid molecules include at least one type of a first primer set having a tag sequence and a base sequence which selectively hybridize with a portion of the DnaJ gene and at least one type of a second primer set which has a tag sequence that is substantially the same as the tag sequence of the first primer set.

10. A solid phase body in which two or more types of nucleic acid molecules selected from the nucleic acid molecules of the nucleic acid molecule kit of claim 8 or 9 have been immobilized.

11. A method of amplifying at least one type of target nucleic acid,
the method comprising a polymerase chain reaction step of performing a polymerase chain reaction using at least one type of a first primer set having a tag sequence and a base sequence which selectively anneals to a portion of the target nucleic acid and at least one type of a second primer set having a tag sequence which is substantially the same as the tag sequence of the first primer set, wherein
the polymerase chain reaction step performs an amplification of the target nucleic acid with the first primer set and an amplification of an amplified product obtained by the amplification with the first primer set with the second primer set, and
the first primer set is directed at, as a target nucleic acid thereof, a portion of a DnaJ gene of a microorganism.

12. The method of amplification of claim 11, wherein the polymerase chain reaction step performs a single PCR reaction cycle.

13. The method of amplification of claim 11 or 12, wherein the second primer set has a primer concentration which is at least 10 times more but not more than 50 times more than a primer concentration of the first primer set.

14. The method of amplification of claim 11, wherein
the first primer set has a primer concentration which is at least 0.005 µM but not more than 0.1 µM,
the second primer set has a primer concentration which is at least 10 times more but not more than 50 times more than the primer concentration of the first primer set,
the amplified product obtained with the second primer set is a nucleic acid of up to 250 bases, and
the polymerase chain reaction step performs a single PCR reaction cycle.

15. The method of amplification of any of claims 11 to 14, wherein the first primer set is directed at, as the target nucleic acid thereof, portions of the DnaJ genes of at least two species of bacteria selected from the genera Staphylococcus, Streptococcus, Serratia, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia.

16. A multiplex PCR-based method for diagnosing pathogenic microorganisms, the pathogenic microorganisms being two or more species selected from the genera Staphylococcus, Streptococcus, Serratia, Klebsiella, Escherichia, Mycobacterium, Legionella, Vibrio, Bacillus, Neisseria, Campylobacter, Chlamydia, Chlamydophila, Mycoplasma, Listeria, Salmonella and Yersinia,
the method comprising:
a polymerase chain reaction step of performing, on a test sample which may contain nucleic acid from the pathogenic microorganisms, a polymerase chain reaction using at least one type of a first primer set having a tag sequence and a base sequence which selectively anneals to a target nucleic acid on a DnaJ gene possessed by the pathogenic microorganism and at least one type of a second primer set having a tag sequence which is substantially the same as the tag sequence of the first primer set; and
a step of detecting an amplified product containing the target nucleic acid.

17. The method of claim 16, wherein the polymerase chain reaction step performs a single PCR reaction cycle.

18. A kit containing primer sets for amplifying, by multiplex PCR, a target nucleic acid of at least one type of pathogenic microorganism, the kit comprising:
a first primer set having a tag sequence and a base sequence which selectively anneals to a target nucleic acid on a DnaJ gene which is possessed by the pathogenic microorganism; and
a second primer set having a tag sequence which is substantially the same as the tag sequence of the first primer set.
